# EUROPEAN PATENT SPECIFICATION

(11) **EP 4 579 398 B1**
(45) Date of publication and mention of the grant of the patent: **28.10.2026**
(21) Application number: 23877509.2
(22) Date of filing: 11.09.2023
(51) Int. Cl.: G06F 3/01, G06F 3/00, G06F 1/16, G06V 40/20, H04W 4/80, A61B 5/11, A61B 5/00

(54) **WEARABLE DEVICE FOR GUIDING USER'S POSTURE AND METHOD THEREOF**
TRAGBARE VORRICHTUNG ZUR FÜHRUNG DER HALTUNG EINES BENUTZERS UND VERFAHREN DAFÜR
DISPOSITIF À PORTER SUR SOI POUR GUIDER LA POSTURE D'UN UTILISATEUR ET PROCÉDÉ ASSOCIÉ

(30) Priority: 11.10.2022 KR 20220130111; 28.10.2022 KR 20220141916
(43) Date of publication of application: 02.07.2025
(73) Proprietor: Samsung Electronics Co., Ltd., Suwon-si, Gyeonggi-do 16677 (KR)
(72) Inventor: OH, Youngjoon, Suwon-si, Gyeonggi-do 16677 (KR); KIM, Sungoh, Suwon-si, Gyeonggi-do 16677 (KR); YEO, Hyungsok, Suwon-si, Gyeonggi-do 16677 (KR); YEOM, Donghyun, Suwon-si, Gyeonggi-do 16677 (KR); LEE, Dasom, Suwon-si, Gyeonggi-do 16677 (KR); LEE, Sanghun, Suwon-si, Gyeonggi-do 16677 (KR)
(74) Representative: HGF
(86) International application number: PCT/KR2023/013605
(87) International publication number: WO 2024/080579

(56) References cited:
- WO-A1-2018/088586
- WO-A1-2022/066350
- KR-A- 20160 143 036
- KR-A- 20200 098 034
- KR-A- 20210 063 723
- KR-A- 20220 102 436
- KR-B1- 101 935 418
- US-A1- 2021 034 222
- US-A1- 2021 327 142

## Description

### [Technical Field]

The following descriptions relate to a wearable device for guiding a user's posture and a method thereof.

### [Background Art]

To provide an enhanced user experience, an electronic device that provides an augmented reality (AR) service displaying information generated by a computer in association with an external object in the real-world, a virtual reality (VR) service to provide an immersive user experience for the virtual world, and/or a mixed reality (MR) service is being developed. The electronic device may be a wearable device that may be worn by a user. For example, the electronic device may be AR glasses.
US 2021327142A1 discloses, according to its abstract, that a computer system enhances guidance information on a display, allowing a user position and orientation in 3D space to be achieved. In some examples, the guidance is visual, for example the blurring of display objects not at the desired position. In some examples, the guidance is aural, for example placing a sound moving to the desired position. In some examples, the guidance is tactile, for example using haptic pads attached to a head-mounted display to push the user in a specified direction. The system may be used to guide the user spatially for the performing of a task, to help the user avoid sensitive components, or to guide the user to position a sensor, such as a camera, in an optimal direction for taking measurements. The system includes head positioning, hand positioning and gaze positioning techniques.
WO 2022066350A1 discloses, according to its abstract, that while head-mountable devices can provide immersive experiences, a user wearing a head-mountable device can also benefit from activities that include body motion and promote the user's health. Because such activities may not be intrinsically necessary to the operation of head-mountable devices, it can be beneficial to provide additional or modified operations that allow a user to continue operation of a head-mountable device while performing the desired body motions. The head-mountable device can detect movement and/or stasis of the user and determine whether motion would be recommended. Upon determining that motion, an additional motion, or a different motion would be beneficial, the head-mountable device can provide an output to the user that promote such motions. The output can include a notification to the user and/or a modification of the user interface that encourages the user to move in a particular way.
WO 2018088586A1 discloses, according to its abstract, a method for managing a neck posture measuring device. The management method for a neck posture measuring device is a method for managing a neck posture measuring device, in which the neck posture measuring device measures the tilted direction and angle of a neck of a user and determines how poor the neck posture of the user is according to the tilted angle of a neck of the user on the basis of the standard posture, the method for managing a neck posture measuring device comprising: a step for receiving information on the tilted direction and angle of a neck of the user from the neck posture measuring device; a step for displaying an avatar according to the tilted direction and angle of a neck of the user; and a step for, when the user inputs the setting regarding the standard posture, setting the tilted direction and angle of a neck of the user at the time of input as the standard posture.
US 2021034222A1 discloses, according to its abstract, that techniques are provided for integrating mobile device and extended reality experiences. Extended reality technologies can include virtual reality (VR), augmented reality (AR), mixed reality (MR), etc. In some examples, a synthetic (or virtual) representation of a device (e.g., a mobile device, such as a mobile phone or other type of device) can be generated and displayed along with VR content being displayed by a VR device (e.g., a head-mounted display (HMD)). In another example, content from the device (e.g., visual content being displayed and/or audio content being played by the device) can be output along with VR content being displayed by the VR device. In another example, one or more images captured by a camera of the device and/or audio obtained by a microphone of the device can be obtained from the device by a virtual reality device and can be output by the virtual reality device.

### [Disclosure]

### [Technical Solution]

The scope of the present invention is determined according to the independent claims. Various embodiments of the present invention are outlined in the dependent claims.

Also disclosed herein is that a wearable device may comprise a sensor, a camera, a display, and a processor. The processor may be configured to obtain, in a state that the wearable device is wom by a user, an image using the camera. The processor may be configured to, in the state, identify a posture of the user based on data of the sensor. The processor may be configured to, based on identifying the posture of the user, which is a first preset posture, obtain a first visual object corresponding to an external object using the camera, and display a second visual object for guiding changing of the posture within a field-of-view (FoV). The processor may be configured to, based on identifying that the posture of the user is changed from the first preset posture to a second preset posture, display the obtained first visual object in an area including a center of the FoV. Also disclosed herein is that a method of a wearable device may comprise obtaining, in a state that the wearable device is wom by a user, an image using a camera. The method of the wearable device may comprise, in the state, identifying a posture of the user based on data of a sensor. The method of the wearable device may comprise, based on identifying the posture of the user, which is a first preset posture, obtaining a first visual object corresponding to an external object using the camera, and displaying a second visual object for guiding changing of the posture within a field-of-view (FoV). The method of the wearable device may comprise, based on identifying that the posture of the user is changed from the first preset posture to a second preset posture, displaying the obtained first visual object in an area including a center of the FoV.

Also disclosed herein is that, in a computer-readable storage medium storing one or more programs according to an example, the one or more programs, when executed by a processor of a wearable device, may cause the processor of the wearable device to obtain, in a state that the wearable device is worn by a user, an image using a camera. The one or more programs, when executed by the processor of the wearable device, may cause the processor of the wearable device to, in the state, identify a posture of the user based on data of a sensor. The one or more programs, when executed by the processor of the wearable device, may cause the processor of the wearable device to, based on identifying the posture of the user, which is a first preset posture, obtain a first visual object corresponding to an external object using the camera, and display a second visual object for guiding changing of the posture within a field-of-view (FoV). The one or more programs, when executed by the processor of the wearable device, may cause the processor of the wearable device to, based on identifying that the posture of the user is changed from the first preset posture to a second preset posture, display the obtained first visual object in an area including a center of the FoV.

### [Description of the Drawings]

FIG. 1 illustrates an example of a block diagram of an electronic device within a network environment, according to one embodiment.
FIG. 2A illustrates an example of a perspective view of a wearable device according to an embodiment.
FIG. 2B illustrates an example of one or more hardware disposed in a wearable device according to an embodiment.
FIG. 3 illustrates an example with respect to a usage state of a wearable device according to an embodiment.
FIG. 4 illustrates an example of a block diagram of a wearable device according to an embodiment.
FIG. 5 illustrates an example in which a wearable device according to an embodiment identifies a posture of a user.
combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

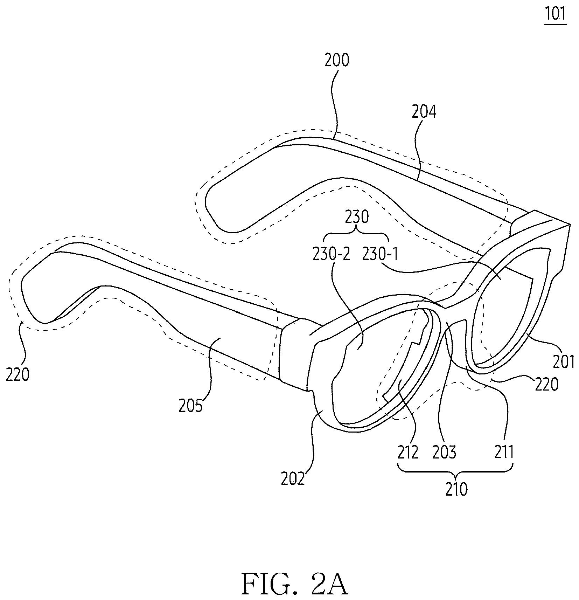

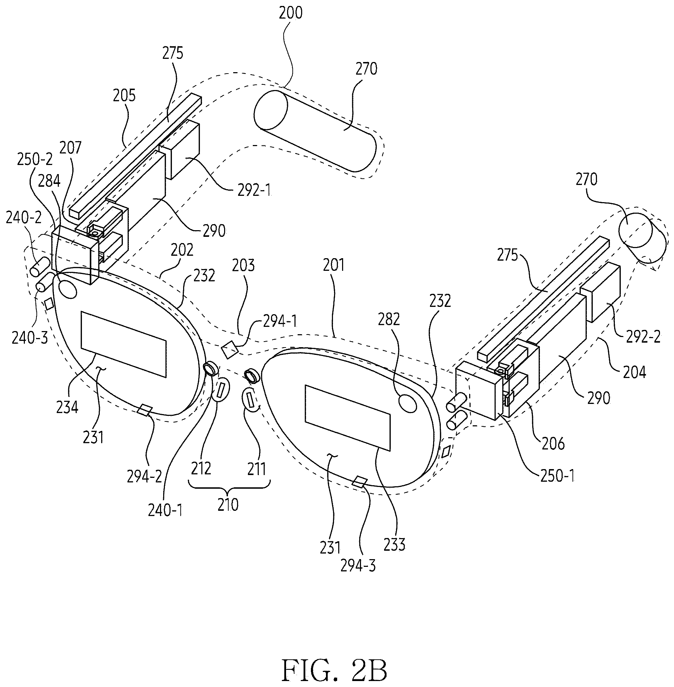

FIG. 2A illustrates an example of a perspective view in a wearable device, according to an embodiment. FIG. 2B illustrates an example of one or more hardware elements disposed in a wearable device, according to an embodiment.

According to an embodiment, the wearable device 101 may be wearable on a portion of the user's body. The wearable device 101 may provide augmented reality (AR), virtual reality (VR), or mixed reality (MR) combining the augmented reality and the virtual reality to a user wearing the wearable device 101. For example, the wearable device 101 may display a virtual reality image provided from at least one optical device 282 and 284 of FIG. 2B on at least one display 230, in response to a user's preset gesture obtained through a motion recognition camera 240-2 of FIG. 2B.

According to an embodiment, the at least one display 230 may provide visual information to a user. For example, the at least one display 230 may include a transparent or translucent lens. The at least one display 230 may include a first display 230-1 and/or a second display 230-2 spaced apart from the first display 230-1. For example, the first display 230-1 and the second display 230-2 may be disposed at positions corresponding to the user's left and right eyes, respectively.

Referring to FIG. 2B, the at least one display 230 may provide visual information transmitted through a lens included in at least one display 230 from ambient light to a user and other visual information distinguished from the visual information. The lens may be formed based on at least one of a fresnel lens, a pancake lens, or a multi-channel lens. For example, the at least one display 230 may include a first surface 231 and a second surface 232 opposite to the first surface 231. A display area may be formed on the second surface 232 of at least one display 230. When the user wears the wearable device 101, ambient light may be transmitted to the user by being incident on the first surface 231 and being penetrated through the second surface 232. For another example, the at least one display 230 may display an augmented reality image in which a virtual reality image provided by the at least one optical device 282 and 284 is combined with a reality screen transmitted through ambient light, on a display area formed on the second surface 232.

According to an embodiment, the at least one display 230 may include at least one waveguide 233 and 234 that transmits light transmitted from the at least one optical device 282 and 284 by diffracting to the user. The at least one waveguide 233 and 234 may be formed based on at least one of glass, plastic, or polymer. A nano pattern may be formed on at least a portion of the outside or inside of the at least one waveguide 233 and 234. The nano pattern may be formed based on a grating structure having a polygonal or curved shape. Light incident to an end of the at least one waveguide 233 and 234 may be propagated to another end of the at least one waveguide 233 and 234 by the nano pattern. The at least one waveguide 233 and 234 may include at least one of at least one diffraction element (e.g., a diffractive optical element (DOE), a holographic optical element (HOE)), and a reflection element (e.g., a reflection mirror). For example, the at least one waveguide 233 and 234 may be disposed in the wearable device 101 to guide a screen displayed by the at least one display 230 to the user's eyes. For example, the screen may be transmitted to the user's eyes based on total internal reflection (TIR) generated in the at least one waveguide 233 and 234.

The wearable device 101 may analyze an object included in a real image collected through a photographing camera 294-1, combine with a virtual object corresponding to an object that become a subject of augmented reality provision among the analyzed object, and display on the at least one display 230. The virtual object may include at least one of text and images for various information associated with the object included in the real image. The wearable device 101 may analyze the object based on a multi-camera such as a stereo camera. For the object analysis, the wearable device 101 may execute time-of-flight (ToF) and/or space recognition (e.g., simultaneous localization and mapping (SLAM)), supported by the multi-camera. The user wearing the wearable device 101 may watch an image displayed on the at least one display 230.

According to an embodiment, a frame 200 may be configured with a physical structure in which the wearable device 101 may be worn on the user's body. According to an embodiment, the frame 200 may be configured so that when the user wears the wearable device 101, the first display 230-1 and the second display 230-2 may be positioned corresponding to the user's left and right eyes. The frame 200 may support the at least one display 230. For example, the frame 200 may support the first display 230-1 and the second display 230-2 to be positioned at positions corresponding to the user's left and right eyes.

Referring to FIG. 2A, according to an embodiment, the frame 200 may include an area 220 at least partially in contact with the portion of the user's body in case that the user wears the wearable device 101. For example, the area 220 of the frame 200 in contact with the portion of the user's body may include an area in contact with a portion of the user's nose, a portion of the user's ear, and a portion of the side of the user's face that the wearable device 101 contacts. According to an embodiment, the frame 200 may include a nose pad 210 that is contacted on the portion of the user's body. When the wearable device 101 is worn by the user, the nose pad 210 may be contacted on the portion of the user's nose. The frame 200 may include a first temple 204 and a second temple 205, which are contacted on another portion of the user's body that is distinct from the portion of the user's body.

For example, the frame 200 may include a first rim 201 surrounding at least a portion of the first display 230-1, a second rim 202 surrounding at least a portion of the second display 230-2, a bridge 203 disposed between the first rim 201 and the second rim 202, a first pad 211 disposed along a portion of the edge of the first rim 201 from one end of the bridge 203, a second pad 212 disposed along a portion of the edge of the second rim 202 from the other end of the bridge 203, the first temple 204 extending from the first rim 201 and fixed to a portion of the wearer's ear, and the second temple 205 extending from the second rim 202 and fixed to a portion of the ear opposite to the ear. The first pad 211 and the second pad 212 may be in contact with the portion of the user's nose, and the first temple 204 and the second temple 205 may be in contact with a portion of the user's face and the portion of the user's ear. The temples 204 and 205 may be rotatably connected to the rim through hinge units 206 and 207 of FIG. 2B. The first temple 204 may be rotatably connected with respect to the first rim 201 through the first hinge unit 206 disposed between the first rim 201 and the first temple 204. The second temple 205 may be rotatably connected with respect to the second rim 202 through the second hinge unit 207 disposed between the second rim 202 and the second temple 205. According to an embodiment, the wearable device 101 may identify an external object (e.g., a user's fingertip) touching the frame 200 and/or a gesture performed by the external object by using a touch sensor, a grip sensor, and/or a proximity sensor formed on at least a portion of the surface of the frame 200.

According to an embodiment, the wearable device 101 may include hardware that performs various functions. For example, the hardware may include a battery module 270, an antenna module 275, the at least one optical device 282 and 284, a speaker 292, a microphone 294, a light emitting module (not illustrated), and/or a printed circuit board 290 (e.g., printed circuit board). Various hardware may be disposed in the frame 200.

According to an embodiment, the microphone 294 of the wearable device 101 may obtain a sound signal, by being disposed on at least a portion of the frame 200. The first microphone 294-1 disposed on the nose pad 210, the second microphone 294-2 disposed on the second rim 202, and the third microphone 294-3 disposed on the first rim 201 are illustrated in FIG. 2B, but the number and disposition of the microphone 294 are not limited to an embodiment of FIG. 2B. In case that the number of the microphone 294 included in the wearable device 101 is two or more, the wearable device 101 may identify a direction of the sound signal by using a plurality of microphones disposed on different portions of the frame 200.

According to an embodiment, the at least one optical device 282 and 284 may project a virtual object on the at least one display 230 in order to provide various image information to the user. For example, the at least one optical device 282 and 284 may be a projector. The at least one optical device 282 and 284 may be disposed adjacent to the at least one display 230 or may be included in the at least one display 230 as a portion of the at least one display 230. According to an embodiment, the wearable device 101 may include a first optical device 282 corresponding to the first display 230-1, and a second optical device 284 corresponding to the second display 230-2. For example, the at least one optical device 282 and 284 may include the first optical device 282 disposed at a periphery of the first display 230-1 and the second optical device 284 disposed at a periphery of the second display 230-2. The first optical device 282 may transmit light to the first waveguide 233 disposed on the first display 230-1, and the second optical device 284 may transmit light to the second waveguide 234 disposed on the second display 230-2.

In an embodiment, a camera 240 may include the photographing camera, an eye tracking camera (ET CAM) 240-1, and/or the motion recognition camera 240-2. The photographing camera, the eye tracking camera 240-1, and the motion recognition camera 240-2 may be disposed at different positions on the frame 200 and may perform different functions. The eye tracking camera 240-1 may output data indicating a position of eye or a gaze of the user wearing the wearable device 101. For example, the wearable device 101 may detect the gaze from an image including the user's pupil obtained through the eye tracking camera 240-1. An example in which the eye tracking camera 240-1 is disposed toward the user's right eye is illustrated in FIG. 2B, but the embodiment is not limited thereto, and the eye tracking camera 240-1 may be disposed alone toward the user's left eye or may be disposed toward two eyes.

In an embodiment, the photographing camera may photograph a real image or background to be matched with a virtual image in order to implement the augmented reality or mixed reality content. The photographing camera may photograph an image of a specific object existing at a position viewed by the user and may provide the image to the at least one display 230. The at least one display 230 may display one image in which a virtual image provided through the at least one optical device 282 and 284 is overlapped with information on the real image or background including an image of the specific object obtained by using the photographing camera. In an embodiment, the photographing camera 294-1 may be disposed on the bridge 203 disposed between the first rim 201 and the second rim 202.

In an embodiment, the eye tracking camera 240-1 may implement a more realistic augmented reality by matching the user's gaze with the visual information provided on the at least one display 230, by tracking the gaze of the user wearing the wearable device 101. For example, when the user looks at the front, the wearable device 101 may naturally display environment information associated with the user's front on the at least one display 230 at a position where the user is positioned. The eye tracking camera 240-1 may be configured to capture an image of the user's pupil in order to determine the user's gaze. For example, the eye tracking camera 240-1 may receive gaze detection light reflected from the user's pupil and may track the user's gaze based on the position and movement of the received gaze detection light. In an embodiment, the eye tracking camera 240-1 may be disposed at a position corresponding to the user's left and right eyes. For example, the eye tracking camera 240-1 may be disposed in the first rim 201 and/or the second rim 202 to face the direction in which the user wearing the wearable device 101 is positioned.

In an embodiment, the motion recognition camera 240-2 may provide a specific event to the screen provided on the at least one display 230 by recognizing the movement of the whole or portion of the user's body, such as the user's torso, hand, or face. The motion recognition camera 240-2 may obtain a signal corresponding to motion by recognizing the user's motion (e.g., gesture recognition), and may provide a display corresponding to the signal to the at least one display 230. The processor may identify a signal corresponding to the operation and may perform a preset function based on the identification. In an embodiment, the motion recognition camera 240-2 may be disposed on the first rim 201 and/or the second rim 202.

The camera 240 included in the wearable device 101 is not limited to the above-described eye tracking camera 240-1 and the motion recognition camera 240-2. For example, the wearable device 101 may identify an external object included in the FoV by using the camera 240 disposed toward the user's FoV. The wearable device 101 identifying the external object may be performed based on a sensor for identifying a distance between the wearable device 101 and the external object, such as a depth sensor and/or a time of flight (ToF) sensor. The camera 240 disposed toward the FoV may support an autofocus function and/or an optical image stabilization (OIS) function. For example, in order to obtain an image including a face of the user wearing the wearable device 101, the wearable device 101 may include the camera 240 (e.g., a face tracking (FT) camera) disposed toward the face.

Although not illustrated, the wearable device 101 according to an embodiment may further include a light source (e.g., LED) that emits light toward a subject (e.g., user's eyes, face, and/or an external object in the FoV) photographed by using the camera 240. The light source may include an LED having an infrared wavelength. The light source may be disposed on at least one of the frame 200, and the hinge units 206 and 207.

According to an embodiment, the battery module 270 may supply power to electronic components of the wearable device 101. In an embodiment, the battery module 270 may be disposed in the first temple 204 and/or the second temple 205. For example, the battery module 270 may be a plurality of battery modules 270. The plurality of battery modules 270, respectively, may be disposed on each of the first temple 204 and the second temple 205. In an embodiment, the battery module 270 may be disposed at an end of the first temple 204 and/or the second temple 205.

In an embodiment, the antenna module 275 may transmit the signal or power to the outside of the wearable device 101 or may receive the signal or power from the outside. The antenna module 275 may be electrically and/or operably connected to the communication circuitry. In an embodiment, the antenna module 275 may be disposed in the first temple 204 and/or the second temple 205. For example, the antenna module 275 may be disposed close to one surface of the first temple 204 and/or the second temple 205.

In an embodiment, the speaker 292 may output a sound signal to the outside of the wearable device 101. A sound output module may be referred to as a speaker. In an embodiment, the speaker 292 may be disposed in the first temple 204 and/or the second temple 205 in order to be disposed adjacent to the ear of the user wearing the wearable device 101. For example, the speaker 292 may include a second speaker 292-2 disposed adjacent to the user's left ear by being disposed in the first temple 204, and a first speaker 292-1 disposed adjacent to the user's right ear by being disposed in the second temple 205.

According to an embodiment, the light emitting module (not illustrated) may include at least one light emitting element. The light emitting module may emit light of a color corresponding to a specific state or may emit light through an operation corresponding to the specific state in order to visually provide information on a specific state of the wearable device 101 to the user. For example, when the wearable device 101 requires charging, it may emit red light at a constant cycle. In an embodiment, the light emitting module may be disposed on the first rim 201 and/or the second rim 202.

Referring to FIG. 2B, according to an embodiment, the wearable device 101 may include the printed circuit board (PCB) 290. The PCB 290 may be included in at least one of the first temple 204 or the second temple 205. The PCB 290 may include an interposer disposed between at least two sub PCBs. On the PCB 290, one or more hardware included in the wearable device 101 may be disposed. The wearable device 101 may include a flexible PCB (FPCB) for interconnecting the hardware.

According to an embodiment, the wearable device 101 may include at least one of a gyro sensor, a gravity sensor, and/or an acceleration sensor for detecting the posture of the wearable device 101 and/or the posture of a body part (e.g., a head) of the user wearing the wearable device 101. Each of the gravity sensor and the acceleration sensor may measure gravity acceleration, and/or acceleration based on preset 3-dimensional axes (e.g., x-axis, y-axis, and z-axis) perpendicular to each other. The gyro sensor may measure angular velocity of each of preset 3-dimensional axes (e.g., x-axis, y-axis, and z-axis). At least one of the gravity sensor, the acceleration sensor, and the gyro sensor may be referred to as an inertial measurement unit (IMU). According to an embodiment, the wearable device 101 may identify the user's motion and/or gesture performed to execute or stop a specific function of the wearable device 101 based on the IMU. FIGS. 2A and 2B illustrate a shape like AR glasses, but are not limited thereto. For example, the wearable device 101 may include an HMD device and/or a visual see-through (VST) device. For example, the VST device may provide a UI for augmented reality (AR) based on a device worn on the user's head, such as the HMD device.

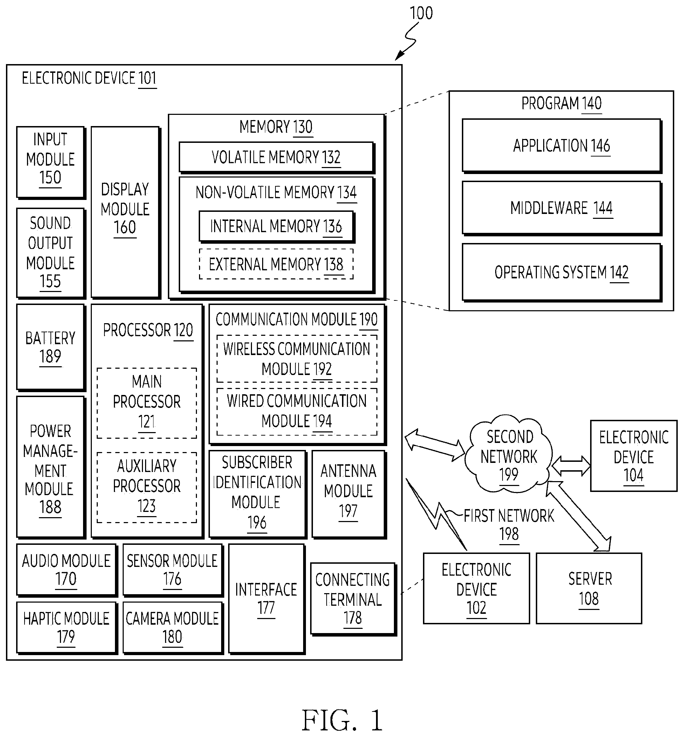

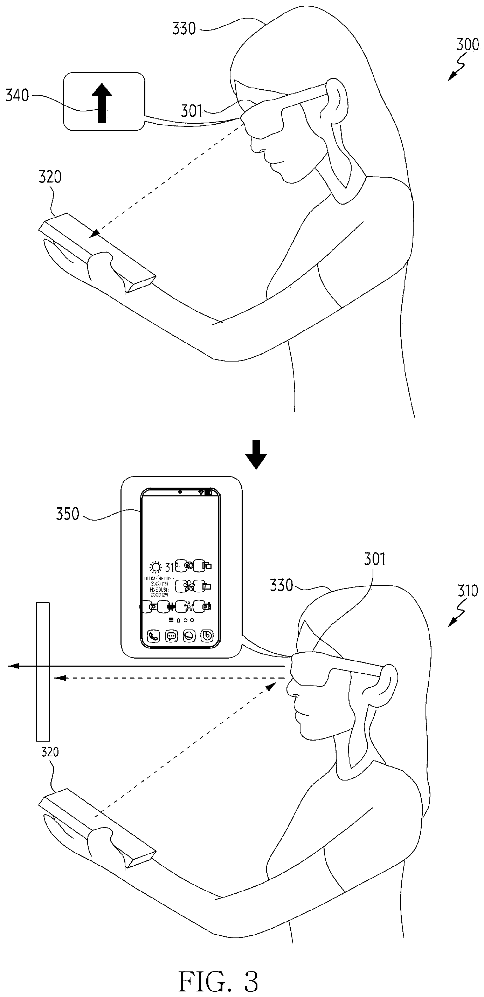

FIG. 3 illustrates an example with respect to a usage state of a wearable device according to an embodiment. A wearable device 301 of FIG. 3 may include the electronic device 101 of FIG. 1. The wearable device 301 of FIG. 3 may include the wearable device 200 of FIG. 2A to FIG. 2B. For example, the wearable device 301 may include AR glasses wearable on a body of a user.

The wearable device 301 according to an embodiment may include a camera disposed toward a front of a user in a state of being worn by the user. The front of the user may include a direction in which a head of the user and/or two eyes included in the head face. In order to provide a user interface (UI) based on AR, VR, and/or mixed reality (MR) to the user wearing the wearable device 301, the wearable device may control the camera. The UI may be associated with a metaverse service provided by the wearable device 301 and/or a server connected to the wearable device 301.

Referring to FIG. 3, the wearable device 301 according to an embodiment may be worn by a user. The wearable device 301 may be worn on at least a portion of a body part (e.g., an ear of a user 330) of the user 330. For example, the wearable device 301 may identify an external object 320 in a state 300 of being worn by the user 330. For example, the wearable device 301 may identify the external object 320, based on at least one sensor (e.g., a time-of-flight (ToF) sensor, a gyro sensor, and/or an accelerometer) included in the wearable device 301. For example, the wearable device 301 may identify the external object 320 based on data of the sensor. The wearable device 301 may identify a posture of the user 330, based on identifying the data of the sensor, and/or the external object 320. For example, the wearable device 301 may identify the external object 320 based on the ToF sensor among the sensors. The wearable device 301 may identify a distance between the external object 320 and the wearable device 301 based on the ToF sensor. For example, the wearable device 301 may detect the external object 320, based on obtaining data matching a parameter less than a first preset distance (e.g., 50 cm) using the ToF sensor. The wearable device 301 may identify the posture of the user 330 of the wearable device 301 based on detecting the external object. The wearable device 301 may identify that the posture of the user 330 corresponds to a preset posture based on the parameter obtained using the ToF sensor.

The wearable device 301 according to an embodiment may identify the distance between the external object 320 and the wearable device 301 using the ToF sensor. The wearable device 301 may obtain a first parameter for identifying the distance. The wearable device 301 may obtain a second parameter different from the first parameter for identifying the posture of the user of the wearable device 301, by using a sensor different from the ToF sensor. The wearable device 301 may identify the posture of the user 330 based on the first parameter and the second parameter. The wearable device 301 may identify the posture of the user 330, based on first data for obtaining the first parameter and second data for obtaining the second parameter. The first data may include data and/or an electrical signal, obtained based on the ToF sensor. The second data may include data and/or an electrical signal, obtained based on a sensor (e.g., the accelerometer, and/or the gyro sensor) different from the ToF sensor.

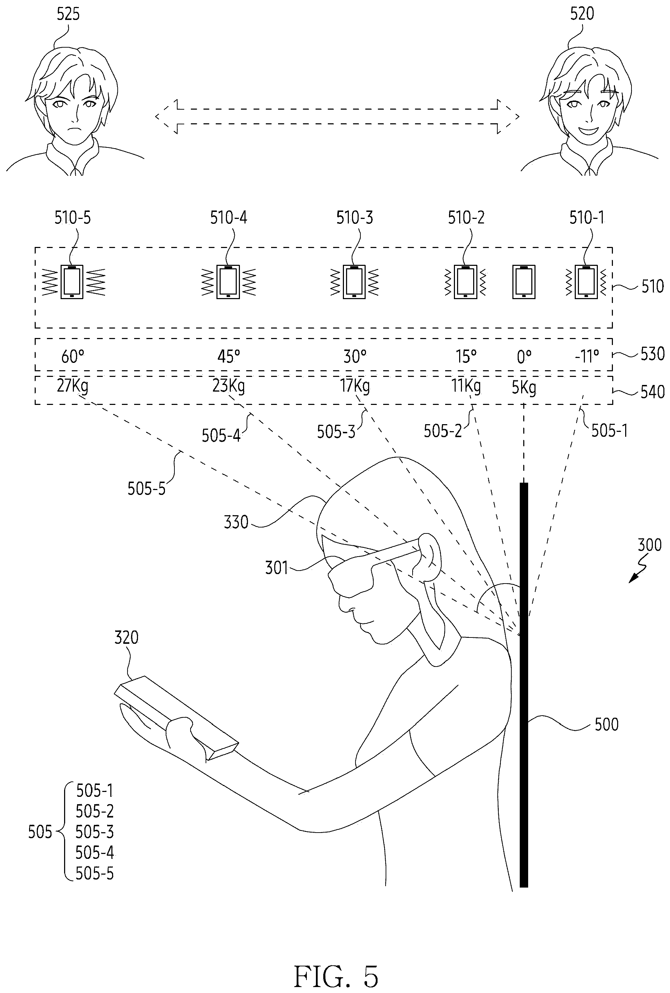

The wearable device 301 according to an embodiment may identify that the posture of the user 330 corresponds to the preset posture using the accelerometer. For example, in the state 300, the wearable device 301 may identify that the posture of the user 330 matches the preset posture. For example, the preset posture may be associated with an angle between a first axis associated with a body part (e.g., a back) of the user 330, and a second axis rotatable according to changing of the posture of the user 330. For example, the first axis may be formed based on the back of the user 330. For example, the second axis may be formed in a direction extending from a neck of the user 330. The wearable device 301 may identify the posture of the user 330 based on the angle between the first axis and the second axis. For example, the posture of the user 330 identified in the state 300 may be the preset posture. For example, the preset posture may be associated with a forward head posture (FHP) or a turtle neck syndrome (or text neck syndrome). A description associated with the preset posture of the user 330 will be described later in FIG. 5.

The wearable device 301 according to an embodiment may identify that the posture of the user 330 matches the preset posture. The wearable device 301 may control a camera based on identifying that the posture of the user 330 matches the preset posture. For example, the wearable device 301 may identify the external object 320 included in a field-of-view (FoV) of the user 330 by controlling the camera. In case of identifying the external object 320, the wearable device 301 may identify that the posture of the user 330 matches the preset posture. The wearable device 301 may obtain a virtual object corresponding to the external object 320 based on identifying the external object 320. The virtual object may include a first visual object 350.

The wearable device 301 according to an embodiment may guide the posture of the user 330 from the state 300 to a state 310. For example, the wearable device 301 may display a second visual object 340 for guiding the posture of the user 330, based on the posture of the user 330 matching the preset posture. For example, in the state 300 in which the external object 320 is identified within the FoV, the wearable device 301 may display the visual object 340 for inducing a gaze of the user 330. For example, the wearable device 301 may display the second visual object 340 for guiding changing of the posture of the user 330 while the external object 320 is identified in the FoV. For example, the visual object may include an arrow for inducing the gaze of the user 330, or an avatar for indicating the posture of the user 330. For example, while displaying the second visual object 340 for inducing a change in the posture of the user 330 in the state 310, the wearable device 301 may capture at least a portion (e.g., a screen of a mobile phone, a screen of a laptop, and a portion where content of a book is displayed) of the external object 320.

**In** the state 310, the wearable device 301 according to an embodiment may cease displaying of the second visual object 320 for guiding changing of the posture of the user 330. In the state 310, the wearable device 301 may cease displaying of the second visual object 320, and display an image associated with at least a portion of the external object 320 captured in the state 300 in the FoV. For example, the wearable device 301 may display the first visual object 350 obtained from the external object 320 in the FoV. For example, the wearable device 301 may use at least one sensor to identify the state 310. For example, the wearable device 301 may identify a parameter matching a second preset distance (e.g., 2 m) obtained using a depth sensor. The wearable device 301 may identify the state 310 based on identifying the parameter matching the second preset distance. The wearable device 301 may identify the state 310 based on the angle between the first axis and the second axis obtained using the accelerometer.

As described above, the wearable device 301 according to an embodiment may identify the posture of the user 330 in the state 300. In the state 300, the wearable device 301 may display a visual object for guiding changing of the posture of the user 330, based on identifying the posture of the user 330. The wearable device 301 may induce changing of the posture of the user 330, by displaying the second visual object 340 for guiding changing of the posture of the user 330. The wearable device 301 may prevent the forward head posture (FHP) or the turtle neck syndrome (or the text neck syndrome) of the user 330, by inducing changing of the posture of the user 330.

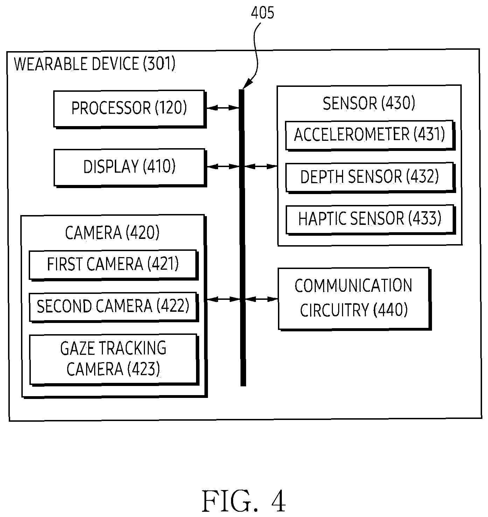

FIG. 4 illustrates an example of a block diagram of a wearable device according to an embodiment. A wearable device 301 of FIG. 4 may include the wearable device 200 of FIG. 2A and/or FIG. 2B and/or the wearable device 301 of FIG. 3. The wearable device 301 according to an embodiment may include a camera disposed toward a front of a user in a state of being worn by the user. The front of the user may include a direction in which a head of the user and/or two eyes included in the head face. In order to provide a user interface (UI) based on AR, virtual reality (VR), and/or mixed reality (MR) to the user wearing the wearable device 301, the wearable device 301 may control the camera. The UI may be associated with a metaverse service provided by the wearable device 301 and/or a server connected to the wearable device 301.

Referring to FIG. 4, the wearable device 301 according to an embodiment may include at least one of a processor 120, a display 410, a camera 420, a sensor 430, or communication circuitry 440. The processor 120, the display 410, the camera 420, the sensor 430, and the communication circuitry 440 may be electronically and/or operably coupled with each other by an electronical component such as a communication bus 405. Hereinafter, hardware being operably coupled may mean that a direct connection or an indirect connection between the hardware is established by wire or wirelessly, so that second hardware among the hardware is controlled by first hardware. Although it is illustrated in different blocks, an embodiment is not limited thereto. A portion (e.g., at least a portion of the processor 120 and the communication circuitry 440) of hardware of FIG. 4 may be included in a single integrated circuit, such as a system on a chip (SoC). A type and/or the number of hardware included in the wearable device 301 is not limited as illustrated in FIG. 4. For example, the wearable device 301 may include only a portion of the hardware illustrated in FIG. 4.

The processor 120 of the wearable device 301 according to an embodiment may include hardware for processing data based on one or more instructions. The hardware for processing data may include, for example, an arithmetic and logic unit (ALU), a floating point unit (FPU), a field programmable gate array (FPGA), a central processing unit (CPU), and/or an application processor (AP). The processor 120 may have a structure of a single-core processor, or may have a structure of a multi-core processor such as a dual core, a quad core, a hexa core, and an octa core.

The display 410 of the wearable device 301 according to an embodiment may output visualized information to a user (e.g., the user 330 of FIG. 3). For example, the display 410 may output the visualized information to the user by being controlled by the processor 120 including circuitry such as a graphic processing unit (GPU). The display 410 may include a flat panel display (FPD) and/or electronic paper. The FPD may include a liquid crystal display (LCD), a plasma display panel (PDP), and/or one or more light emitting diodes (LEDs). The LED may include an organic LED (OLED).

The communication circuitry 440 of the wearable device 301 according to an embodiment may include a hardware component for supporting transmission and/or reception of an electrical signal between the wearable device 301 and an external electronic device. For example, the communication circuitry 440 may include at least one of a MODEM, an antenna, and an optic/electronic (O/E) converter. The communication circuitry 440 may support the transmission and/or the reception of the electrical signal, based on various types of protocols such as Ethernet, a local area network (LAN), a wide area network (WAN), a wireless fidelity (WiFi), Bluetooth, bluetooth low energy (BLE), ZigBee, a long term evolution (LTE), and a 5th generation (5G) new radio (NR).

According to an embodiment, the camera 420 of the wearable device 301 may include a first camera 421 looking at a direction matching a gaze of the user, a second camera 422 looking at a direction below by a preset angle (e.g., 15 degrees) based on the gaze of the user, and/or a gaze tracking camera 423 for tracking the gaze of the user. According to an embodiment, the camera 420 of the wearable device 301 may include one or more optical sensors (e.g., a charged coupled device (CCD) sensor and a complementary metal oxide semiconductor (CMOS) sensor) that indicate an electrical signal indicating a color and/or brightness of light. A plurality of optical sensors included in the camera 420 may be disposed in a form of a 2 dimensional array. The camera 420 may generate 2D frame data corresponding to light reaching the optical sensors of the 2 dimensional array, by obtaining an electrical signal of each of the plurality of optical sensors substantially simultaneously. For example, image data captured using the camera 420 may mean one 2D frame data obtained from the camera 420. For example, video data captured using the camera 420 may mean a sequence of a plurality of 2D frame data obtained from the camera 420 according to a frame rate. The camera 420 may further include a flashlight, disposed toward a direction in which the camera 420 receives light, for outputting light toward the direction. Although the camera 420 is illustrated based on a single block, the number of cameras 420 included in the wearable device 301 is not limited to the embodiment. The camera 420 according to an embodiment may be disposed toward a FoV of the user in a state that the user wears the wearable device 301. The processor 120 may obtain frames including a scene of the FoV by controlling the camera 420 disposed toward the FoV. For example, the camera 420 disposed toward the FoV may include the first camera 421. Based on the camera 420 disposed toward the FoV, the wearable device 301 may obtain an image including at least a portion of an external object (e.g., the external object 320 of FIG. 3) included in the FoV. The gaze tracking camera 423 of the wearable device 301 according to an embodiment may be a camera for tracking the gaze of the user. The gaze tracking camera 423 may be disposed in a direction facing the user. The wearable device 301 may track the gaze of the user based on the gaze tracking camera 423. The wearable device 301 may identify an external object in a direction in which the user is looking at, based on tracking the gaze of the user. The wearable device 301 may identify a region of interest (ROI) that is at least a portion of the external object, based on identifying the external object in the direction the user is looking at. The wearable device 301 may display an image associated with the ROI in the FoV, based on identifying the ROI.

The wearable device 301 according to an embodiment may obtain an image using the second camera 422. For example, the second camera 422 may have a different FoV from the first camera 421. For example, the wearable device 301 may obtain an image in a direction looking downward by the preset angle (e.g., 15 degrees) from a direction in which the display is looking at, using the second camera 422. For example, when the user looks at the front, the wearable device 301 may obtain an image including an external object using the second camera 422. The wearable device 301 may render the external object included in the image obtained by using the second camera 422. The wearable device 301 may display a virtual object (e.g., the first visual object 350 of FIG. 3) corresponding to the rendered external object in the FoV based on rendering the external object.

The sensor 430 of the wearable device 301 according to an embodiment may include an accelerometer 431, a depth sensor 432, and/or a haptic sensor 433. The accelerometer 431 of the wearable device 301 may output electrical information indicating magnitude of acceleration of gravity measured at each of a plurality of preset axes (e.g., an x-axis, a y-axis, and a z-axis) perpendicular to each other. For example, the processor 120 of the wearable device 301 may detect a posture of the user in a physical space based on the electrical information outputted from the accelerometer 431. The motion detected by the wearable device 301 may indicate an orientation of the wearable device 301 detected by the accelerometer 431. For example, based on the accelerometer 431, the wearable device 301 may identify an angle between a first axis associated with a body part of the user, and a second axis rotatable based on the first axis according to changing of the posture of the user. For example, the processor 120 of the wearable device 301 may obtain electrical information associated with the second axis using the accelerometer 431. The wearable device 301 may identify the posture of the user based on the electrical information associated with the second axis.

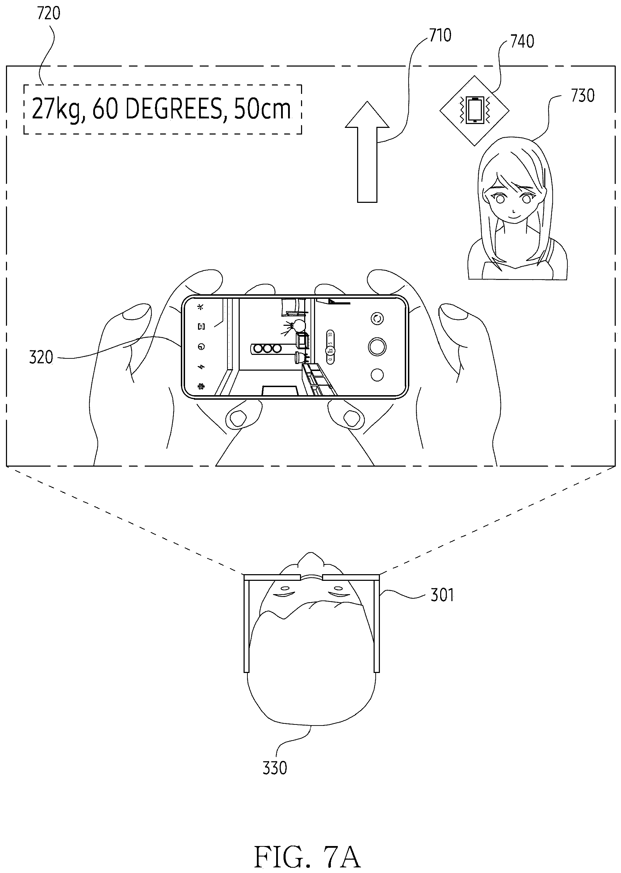

The depth sensor 432 of the wearable device 301 according to an embodiment may output electrical information associated with light outputted from the depth sensor 432 being reflected and received by the outside. For example, the wearable device 301 may identify a distance between the wearable device 301 and an external object based on the reflection of the light. For example, the depth sensor 432 may include a time of flight (ToF) sensor, structured light, and a light detection and ranging (LiDAR). The wearable device 301 may identify the posture of the user of the wearable device 301 based on identifying the distance between the wearable device 301 and the external object, which is a first preset distance (e.g., 50 cm). The wearable device 301 may display a visual object for guiding changing of the posture in the FoV, based on identifying that the posture is a preset posture (e.g., FHP). The operation of displaying the visual object for guiding changing of the posture will be described later in FIG. 7A to FIG. 7B.

The haptic sensor 433 of the wearable device 301 according to an embodiment may be a sensor for converting an electrical signal into a physical force. For example, the wearable device 301 may control the haptic sensor 433 based on identifying the posture of the user, which is the preset posture. For example, the wearable device 301 may output vibration using the haptic sensor 433, in order to guide changing of the posture of the user, which is the preset posture. For example, the wearable device 301 may output intensity of the vibration differently according to the posture of the user. The operation of outputting the intensity of the vibration differently according to the posture of the user will be described later in FIG. 5.

As described above, the wearable device 301 according to an embodiment may identify the posture of the user based on data of the sensor 430 in the state of being worn by the user. The wearable device 301 may identify that the posture of the user matches the preset posture. The wearable device 301 may identify an external object included in the FoV by using the camera 420 based on identifying that the posture of the user matches the preset posture. For example, the wearable device 301 may identify the external object included in the FoV using the first camera 421. The wearable device 301 may display a first visual object corresponding to the identified external object in the FoV through the display 410, based on the identified external object. The wearable device 301 may display the first visual object in an area including a center of the FoV. While displaying the first visual object in the area including the center of the FoV, the wearable device 301 may display a second visual object (e.g., a shape indicating a direction) for guiding changing of the posture of the user. The wearable device 301 may prevent the forward head posture or a turtle neck syndrome of the user of the wearable device 301 by displaying the first visual object and the second visual object in the FoV.

FIG. 5 illustrates an example in which a wearable device according to an embodiment identifies a posture of a user. A wearable device 301 of FIG. 5 may include the wearable device 200 of FIG. 2A and/or FIG. 2B, and the wearable device 301 of FIG. 3 and/or FIG. 4.

Referring to FIG. 5, the wearable device 301 according to an embodiment may identify a posture of a user 330 of the wearable device 301. For example, the wearable device 301 may identify the posture of the user 330 based on a sensor included in the wearable device 301. The wearable device 301 may identify the posture of the user 330 based on data of the sensor.

The wearable device 301 according to an embodiment may identify the posture of the user 330 based on data of an accelerometer (e.g., the accelerometer 431 of FIG. 4). The wearable device 301 may identify an angle between a first axis 500 associated with a body part of the user and a second axis 505 rotatable based on the first axis. The wearable device 301 may identify the posture of the user 330 based on identifying the angle. For example, the wearable device 301 may identify the angle based on data obtained by using the accelerometer. The wearable device 301 may identify the posture of the user 330 based on a parameter associated with the data obtained by using the accelerometer. For example, the wearable device 301 may obtain a parameter 540 associated with magnitude of a force, or a load, applied to a body part (e.g., a neck of the user) of the user. For example, the wearable device 301 may obtain the parameter 540 associated with the load based on the angle. For example, the parameter 540 associated with the load may be referred to by Equation 1 below. [Equation 1] $y = 5 + \left|0.4\times x\right|$

In the Equation 1, y may mean the parameter 540. In the Equation 1, x may mean an angle obtained based on the accelerometer. In the Equation 1, '5' and/or '0.4' may be arbitrary values. The wearable device 301 may identify the posture of the user 330 based on obtaining the parameter 540. The wearable device 301 may execute an operation for guiding changing of the posture based on identifying the posture of the user 330. For example, the wearable device 301 may display a second visual object for guiding changing of the posture of the user 330. A description associated with the second visual object will be described later in FIG. 7A to FIG. 7B.

The wearable device 301 according to an embodiment may display a third visual object 520 or 525 associated with the posture of the user 330 in a FoV based on identifying the posture of the user 330. For example, the wearable device 301 may identify a second axis 505-2 inclined by 0 to 11 degrees based on a first axis 500. The wearable device 301 may identify a second axis 505-1 inclined by 0 to -11 degrees based on the first axis 500. The wearable device 301 may display the third visual object 520 in the FoV based on identifying the second axis 505-1 or 505-2. The third visual object 520 may be associated with the posture of the user 330. The third visual object 520 may be displayed when the posture of the user 330 is a correct posture. For example, while changing from the second axis 505-1 identified as -11 degrees to a second axis 505-5 identified as 60 degrees, the wearable device 301 may change from the third visual object 520 to the third visual object 525, in association with the second axis 505-1 to the second axis 505-5. For example, the third visual object may be associated with a facial expression of an avatar. For example, the facial expression of the avatar may change while changing from the second axis 505-1 to the second axis 505-5. For example, the facial expression of the avatar may be a smiling facial expression in a state that the angle between the first axis 500 and the second axis 505 is -11 degrees to 11 degrees. For example, the facial expression of the avatar may be a frown facial expression in a state that the angle between the first axis 500 and the second axis 505 is greater than 11 degrees.

Based on identifying the angle between the first axis 500 and the second axis 505, the wearable device 301 according to an embodiment may display a fourth visual object 530 for indicating the angle in the FoV. For example, the wearable device 301 may display, in the FoV, the fourth visual object 530 for indicating a parameter associated with the angle obtained based on the accelerometer and/or a gyro sensor. The wearable device 301 may display the fourth visual object 530 matching the parameter in the FoV. For example, based on identifying an angle between the first axis 500 and the second axis 505-3 as 30 degrees, the wearable device 301 may display the fourth visual object 530 for indicating the 30 degrees in the FoV.

The wearable device 301 according to an embodiment may control a haptic sensor (e.g., the haptic sensor 433 of FIG. 4) based on identifying the posture of the user 330. The wearable device 301 may control intensity of vibration generated by the haptic sensor. For example, the wearable device 301 may display a fifth visual object 510 matching the intensity of the vibration generated by the haptic sensor in the FoV. For example, the wearable device 301 may control the haptic sensor with the intensity of the vibration matching the fifth visual object 510. For example, based on identifying the second axis 505-5 forming 60 degrees based on the first axis 500, the wearable device 301 may provide vibration feedback of a relatively stronger intensity than identifying a second axis 505-4 to the second axis 505-1 forming 45 degrees to -11 degrees based on the first axis 500. The wearable device 301 may guide changing of the posture of the user of the wearable device 301 by providing the vibration feedback.

The wearable device 301 according to an embodiment may identify the posture of the user 330, based on a threshold of the parameter associated with the angle obtained by the accelerometer. For example, the wearable device 301 may identify the angle, which is greater than a first threshold (e.g., -11 degrees) and less than a second threshold (e.g., 11 degrees) greater than the first threshold. Based on identifying the angle, which is greater than the first threshold (e.g., - 11 degrees) and less than the second threshold (e.g., 11 degrees) greater than the first threshold, the wearable device 301 may identify a first preset posture (e.g., a posture in which the posture of the user 330 of the wearable device 301 is in a normal range). For example, based on identifying the angle, which is less than or equal to the first threshold, the wearable device 301 may identify a second preset posture (e.g., a posture in which the neck of the user 330 is tilted back). The wearable device 301 may provide the vibration feedback to the user 330 by controlling the haptic sensor based on identifying the second preset posture. The wearable device 301 may guide changing of the posture of the user 330 of the wearable device 301 by executing the operation. For example, the wearable device 301 may identify an angle, which is greater than or equal to the second threshold. Based on identifying the angle, which is greater than or equal to the second threshold, the wearable device 301 may identify a third preset posture (e.g., a posture in which the neck of the user 330 is bent forward) of the user 330. Based on identifying the third preset posture, the wearable device 301 may display a visual object for inducing the user 330 to change the posture to the first preset posture. Based on identifying the third preset posture, the wearable device 301 may provide the vibration feedback for inducing the user 330 to change the posture to the first preset posture.

As described above, the wearable device 301 according to an embodiment may identify the posture of the user 330 based on data of the sensor. For example, the wearable device 301 may identify the posture of the user 330 based on a parameter associated with the data of the sensor. The wearable device 301 may display a visual object for guiding changing of the posture of the user 330 based on identifying the posture of the user 330. Based on identifying the posture of the user 330, the wearable device 301 may provide vibration feedback. The wearable device 301 may guide the posture of the user 330 of the wearable device 301 by displaying the visual object or providing the vibration feedback. The wearable device 301 may prevent a forward head posture or a turtle neck syndrome of the user 330 by guiding the posture of the user 330.

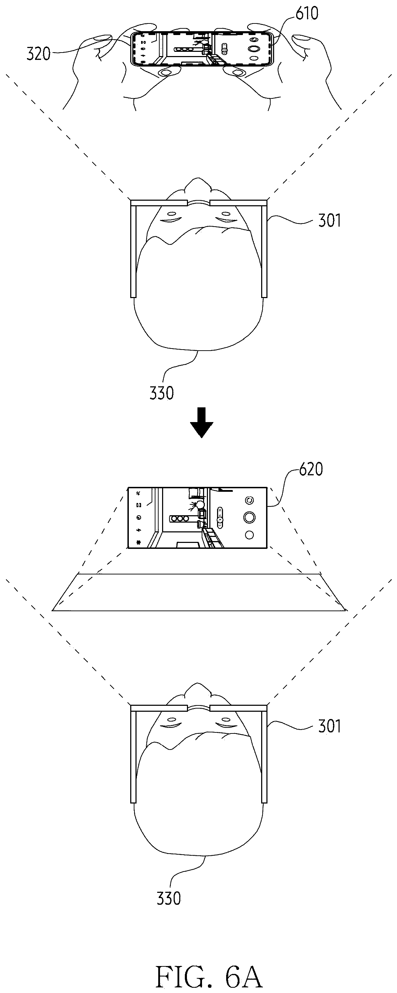

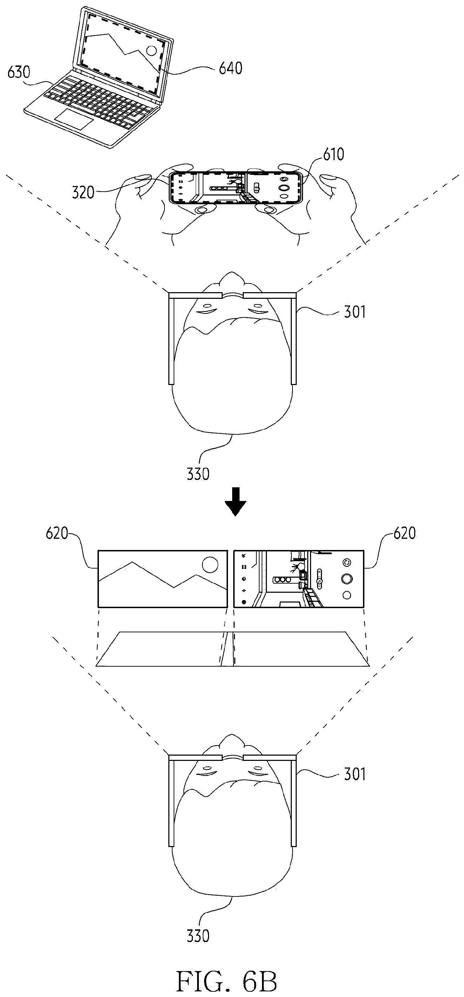

FIG. 6A illustrates an example of a screen displayed in a FoV of a wearable device identifying an external object according to an embodiment. FIG. 6B illustrates an example of a screen displayed in a FoV of a wearable device identifying an external object according to an embodiment. A wearable device 301 of FIG. 6A to FIG. 6B may include the wearable device 200 of FIG. 2A and/or FIG. 2B, and the wearable device 301 of FIG. 3, FIG. 4, and/or FIG. 5.

Referring to FIG. 6A and FIG. 6B, the wearable device 301 according to an embodiment may identify an external object 320 or 630 in the FoV using a camera (e.g., the camera 420 of FIG. 4) in a state of being worn by a user 330. Based on identifying the external object 320 or 630, the wearable device 301 may display a screen associated with the external object 320 or 630.

Referring to FIG. 6A, the wearable device 301 according to an embodiment may identify the external object 320 in the FoV. Based on identifying the external object 320, the wearable device 301 may identify an ROI 610 including at least a portion (e.g., a screen of the external object 320, or an area including text in case that the external object 320 includes the text such as a book) of the external object 320. In the ROI 610, corners of the ROI 610 may not be 90 degrees, such as a trapezoid or a rhombus. Based on identifying the ROI 610, the wearable device 301 may generate an image including the ROI 610. Based on generating the image, the wearable device 301 may change the image. For example, the image may be an image 620 in which the ROI 610 corrects the corners at 90 degrees, such as a rectangle or a square. The wearable device 301 may display the corrected image 620 in the FoV. The wearable device 301 according to an embodiment may obtain information for displaying a screen matching the external object 320 including the text from an external electronic device (e.g., a server) and/or memory of the wearable device 301. For example, the information may be associated with an e-book.

In case that the external object 320 includes communication circuitry like the external electronic device, the wearable device 301 according to an embodiment may establish a communication link with the external object 320. Based on establishing the communication link, the wearable device 301 may request the information for displaying the screen of the external object 320 from the external object 320. The wearable device 301 may receive the information for displaying the screen from the external object 320. Based on receiving the information from the external object 320, the wearable device 301 may display a screen associated with the information. For example, displaying the screen associated with the information may be referred to as a mirroring view function.

The wearable device 301 according to an embodiment may identify the external object 320 including the text, such as the book. For example, based on identifying the external object 320 including the text, the wearable device 301 may identify data stored in an external electronic device (e.g., a server) different from the external object 320, and/or the wearable device 301. For example, the wearable device 301 may receive data associated with the external object 320 from the external electronic device. For example, the wearable device 301 may identify whether the text included in the external object 320 matches the data. The wearable device 301 may identify whether at least a portion of the data and at least a portion of the text included in the external object 320 match. The wearable device 301 may display a screen associated with the data based on identifying whether the matching occurred. For example, identifying whether the matching occurred may include an operation in which a 100 x 100 matrix generated by the wearable device 301 based on the ROI 610, and the data match. The wearable device 301 may identify information (or data) indicating pixels of the ROI 610. The wearable device 301 may generate a 100 x 100 matrix based on the information indicating the pixels of the ROI 610. For example, identifying whether the matching occurred may include an operation of identifying that the 100 x 100 matrix and the data match by a preset ratio. Based on identifying that the 100 x 100 matrix and the data match, the wearable device 301 may display the screen associated with the data in the FoV.

Referring to FIG. 6B, the wearable device 301 according to an embodiment may identify a first external object 320 in a state of being worn by the user 330. The wearable device 301 may identify a second external object 630 in the state of being worn by the user 330. The wearable device 301 may identify a plurality of external objects 320 and 630 in the state of being worn by the user 330. Although two external objects 320 and 630 are illustrated in FIG. 6B, the number of a plurality of external objects identified by the wearable device 301 is not limited.

Based on identifying the first external object 320, the wearable device 301 according to an embodiment may identify a first ROI 610 including at least a portion of the first external object 320. Based on identifying the second external object 630, the wearable device 301 may identify a second ROI 640 including at least a portion of the second external object 630. Based on identifying the plurality of external objects 320 and 630, the wearable device 301 may identify a plurality of ROIs 610 and 640 corresponding to each of the plurality of external objects 320 and 630. Based on identifying the plurality of ROIs 610 and 640, the wearable device 301 may obtain images including the plurality of ROIs 610 and 640. Based on obtaining the images, the wearable device 301 may change (or correct) the images. Based on changing the images, the wearable device 301 may display the changed images 620 and 650 in the FoV. The wearable device 301 may display a first image 620 among the changed images in a first preset area (e.g., a right side of the FoV). The wearable device 301 may display a second image 650 among the changed images in a second preset area (e.g., a left side of the FoV).

The wearable device 301 according to an embodiment may identify a plurality of objects 320 and 630. In case that the plurality of objects 320 and 630 are external electronic devices different from the wearable device 301, the wearable device 301 may establish a communication link with the plurality of objects 320 and 630 through the communication circuitry. Based on establishing the communication link with the external objects 320 and 630, the wearable device 301 may transmit a signal for requesting information associated with a screen of the external objects 320 and 630. The external objects 320 and 630 that have received the signal may transmit the information associated with the screen to the wearable device 301. Based on receiving the information, the wearable device 301 may display screens displayed in a display of the external objects 320 and 630 in the FoV. The wearable device 301 may display the screens in preset areas. For example, the preset areas may include the first preset area and/or the second preset area.

As described above, the wearable device 301 according to an embodiment may identify the external object 320. Based on identifying the external object 320, the wearable device 301 may identify the ROI 610 including at least a portion of the external object 320. Based on identifying the ROI 610, the wearable device 301 may display a screen associated with the ROI 610. The wearable device 301 may display the screen associated with the ROI 610 in a state that the external object 320 is not identified in the FoV. The wearable device 301 may enhance a user 330 experience of the wearable device 301 by displaying the screen. The wearable device 301 may prevent a forward head posture or a turtle neck syndrome of the user 330 by displaying the screen.

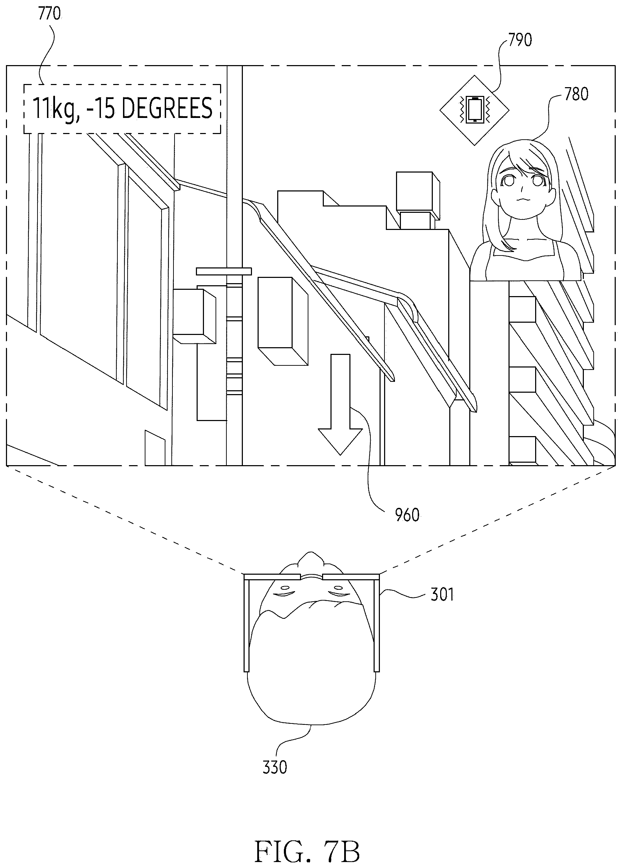

FIG. 7A illustrates an example of a screen displayed in a FoV of a wearable device according to an embodiment. FIG. 7B illustrates an example of a screen displayed in a FoV of a wearable device according to an embodiment. A wearable device 301 of FIG. 7A to FIG. 7B may include the wearable device 200 of FIG. 2A and/or FIG. 2B, and the wearable device 301 of FIG. 3, FIG. 4, FIG. 5, FIG. 6A, and/or FIG. 6B.

Referring to FIG. 7A, the wearable device 301 according to an embodiment may identify a posture of a user 330 in a state of being worn by the user 330. The wearable device 301 may identify that the posture of the user 330 is a first preset posture. For example, the first preset posture may be a posture in which a neck of the user 330 is bent. Based on identifying that the posture of the user 330 corresponds to a preset posture, the wearable device 301 may identify an external object 320 included in the FoV of the user 330 using a camera (e.g., the camera 420 of FIG. 4). Based on identifying the external object 320, the wearable device 301 may display a first visual object corresponding to the external object 320. For example, the first visual object may include the corrected image 620 of FIG. 6A. The wearable device 301 may display the first visual object in an area including a center of the FoV. The wearable device 301 may identify the posture of the user 330 while displaying the first visual object in the area including the center of the FoV. While displaying the first visual object, the wearable device 301 may display a second visual object 710 for guiding changing of the posture of the user 330. For example, the second visual object 710 may be a visual object indicating a preset direction, such as an arrow pointing an upward direction, in the FoV.

The wearable device 301 according to an embodiment may obtain data of a sensor (e.g., the sensor 430 of FIG. 4). The wearable device 301 may display a third visual object 720 for indicating a parameter associated with the data obtained by the sensor. For example, the wearable device 301 may display an angle between a first axis (e.g., the first axis 500 of FIG. 5) associated with a body part of the user 330, and a second axis (e.g., the second axis 505 of FIG. 5) rotatable based on the first axis according to changing of the posture of the user 330 in the third visual object 720. For example, the wearable device 301 may display a parameter associated with a load applied to the neck of the user 330 in the third visual object 720. For example, the wearable device 301 may display a parameter associated with a distance between the wearable device 301 and the external object 320 in the third visual object 720.

The wearable device 301 according to an embodiment may guide changing of the posture of the user 330 using a haptic sensor (e.g., the haptic sensor 433 of FIG. 4). For example, the wearable device 301 may provide vibration feedback based on the posture of user 330. The wearable device 301 may guide changing of the posture of the user 330 using different intensity of the vibration based on the posture of the user 330. The wearable device 301 may display a fourth visual object 740 while guiding changing of the posture based on the haptic sensor. For example, the fourth visual object 740 may indicate the intensity of the vibration.

The wearable device 301 according to an embodiment may display a fifth visual object 730 corresponding to the posture of the user 330. For example, the wearable device 301 may change displaying of the fifth visual object 730 based on the angle between the first axis and the second axis, which is obtained based on the sensor. The fifth visual object 730 may include an avatar corresponding to the posture of the user 330. For example, the fifth visual object 730 may be displayed as an avatar looking at a downward direction.

Referring to FIG. 7B, the wearable device 301 according to an embodiment may identify the posture of the user 330 in the state of being worn by the user 330. For example, the wearable device 301 may identify that the posture of user 330 is a second preset posture. For example, the second preset posture may be a posture in which the neck of the user 330 is tilted. For example, based on identifying the second preset posture, the wearable device 301 may display a second visual object 760 for guiding changing of the posture of the user 330. For example, the second visual object 760 may be a visual object indicating a preset direction, such as an arrow pointing downward direction, in the FoV.

The wearable device 301 according to an embodiment may obtain data of a sensor. The wearable device 301 may display a third visual object 770 for indicating a parameter associated with the data obtained by the sensor. For example, the wearable device 301 may display the angle between the first axis and the second axis in the third visual object 770. For example, the wearable device 301 may display the parameter associated with the load applied to the neck of the user 330 in the third visual object 770.

The wearable device 301 according to an embodiment may guide changing of the posture of the user 330 using the haptic sensor. For example, the wearable device 301 may provide vibration feedback using the haptic sensor based on the posture of the user 330. While providing the vibration feedback, the wearable device 301 may display a fourth visual object 790 for indicating that the vibration feedback is being executed.

The wearable device 301 according to an embodiment may display a fifth visual object 780 corresponding to the posture of the user 330. For example, the wearable device 301 may display the fifth visual object 780 based on the angle between the first axis and the second axis. For example, the fifth visual object 780 may be displayed as an avatar looking in an upward direction.

As described above, the wearable device 301 according to an embodiment may display the visual objects 710, 720, 730, 740, 750, 760, 770, and 780 based on the posture of the user 330. The wearable device 301 may guide changing of the posture of the user 330 by displaying the visual objects 710, 720, 730, 740, 750, 760, 770, and 780. The wearable device 301 may prevent a forward head posture or a turtle neck syndrome of the user 330 by guiding changing of the posture of the user 330.

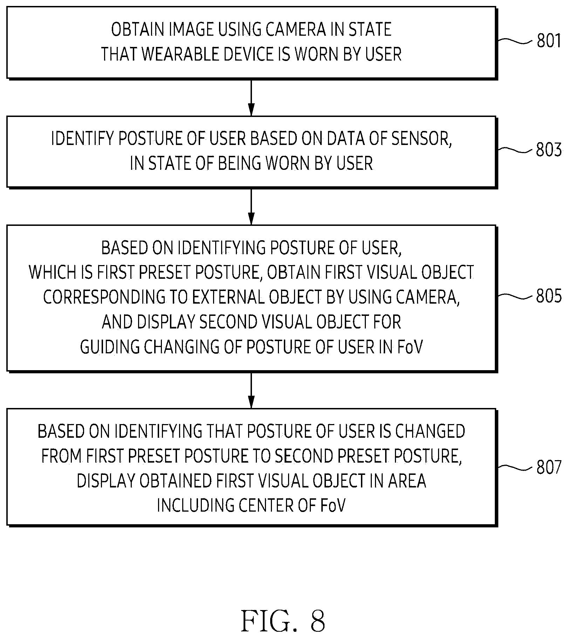

FIG. 8 illustrates an example of a flowchart with respect to an operation of a wearable device according to an embodiment. A wearable device of FIG. 8 may include the wearable device 200 of FIG. 2A and/or FIG. 2B, and the wearable device 301 of FIG. 3, FIG. 4, FIG. 5, FIG. 6A, FIG. 6B, FIG. 7A, and/or FIG. 7B.

Referring to FIG. 8, in operation 801, the wearable device according to an embodiment may be worn by a user. The wearable device may obtain an image using a camera (e.g., the camera 420 of FIG. 4 or the first camera 421 of FIG. 4) in a state of being worn by the user. The wearable device may obtain data (or an electrical signal) based on a sensor included in the wearable device in the state of being worn by the user.

In operation 803, the wearable device according to an embodiment may identify a posture of the user based on a sensor (e.g., the sensor 430 of FIG. 4) in the state of being worn by the user. For example, the wearable device may identify the posture of the user based on data obtained using an accelerometer. For example, the wearable device may identify the posture of the user based on data obtained using a gyro sensor. For example, the wearable device may identify the posture of the user based on data obtained using a depth sensor. For example, the wearable device may identify a distance between an external object and the wearable device using the depth sensor. Based on identifying the distance, the wearable device may identify the posture of the user.

In operation 805, the wearable device according to an embodiment may identify that the posture of the user is a first preset posture. For example, the first preset posture may include a posture in which a neck of the user is bent. For example, the first preset posture may include parameters obtained from a sensor identified as a preset parameter. For example, based on identifying that the posture of the user corresponds to the first preset posture, the wearable device may identify the external object included in a FoV of the user using the camera. Based on identifying the external object, the wearable device may obtain a first visual object (e.g., the first visual object 350 of FIG. 3) corresponding to the external object. The first visual object may be obtained based on rendering the external object. For example, the first visual object may include an image including at least a portion of the external object. For example, the external object may include an electronic device and/or a non-electronic device, such as a book, a laptop, and a mobile phone.

The wearable device according to an embodiment may display a second visual object (e.g., the second visual object 340 of FIG. 3) for guiding changing of the posture of the user. For example, based on identifying the posture of the user corresponding to the first preset posture, the wearable device may display the second visual object in the FoV. For example, the second visual object may include a visual object for inducing a gaze of the user, such as an arrow.

The wearable device according to an embodiment may obtain the first visual object, and display the second visual object in the FoV. The wearable device may obtain the first visual object corresponding to the external object using the camera based on identifying the posture of the user, which is the first preset posture, and display the second visual object for guiding changing of the posture of the user in the FoV.

The wearable device according to an embodiment may obtain the first visual object corresponding to the external object using the camera. Based on obtaining the first visual object, the wearable device may display the first visual object in the FoV. The wearable device may display the first visual object in a preset area (e.g., an area adjacent to a periphery of the FoV) in the FoV. The wearable device according to an embodiment may adjust an alpha value of the first visual object. For example, the alpha value may mean a transparency of the first visual object. The wearable device may display the first visual object based on adjusting the alpha value of the first visual object. The wearable device may display the first visual object in which the alpha value is adjusted in the preset area of the FoV.

In operation 807, the wearable device according to an embodiment may display the first visual object in an area including a center of the FoV. The wearable device may identify that the posture of the user is changed from the first preset posture to a second preset posture. The wearable device may display the first visual object obtained in the operation 805 based on identifying the posture of the user changed from the first preset posture to the second preset posture. For example, the wearable device may display the first visual object in the area including the center of the FoV. The wearable device may display the obtained first visual object in the area including the center of the FoV, based on identifying that the posture of the user is changed from the first preset posture to the second preset posture.

The wearable device according to an embodiment may display the first visual object based on identifying the posture of the user identified as the second preset posture. The wearable device may obtain an image using a second camera, which is different from the camera of the operation 805 that is a first camera, while displaying the first visual object. For example, the second camera may be disposed to form a preset angle (e.g., 15 degrees) with a direction in which the first camera faces. For example, the second camera may be a camera for obtaining an image including an external object. For example, the wearable device may obtain the image using the second camera based on identifying the posture of the user, which is the second preset posture. The wearable device may identify the external object included in the image obtained using the second camera. Based on identifying the external object, the wearable device may generate a third visual object, which is a visual object corresponding to the external object. The wearable device may display the third visual object obtained through the second camera.

As described above, the wearable device according to an embodiment may identify the posture of the user in the state of being worn by the user. The wearable device may identify that the posture of the user is the preset posture. Based on identifying the posture of the user, which is the preset posture, the wearable device may display a visual object for guiding changing of the posture of the user. The wearable device may prevent a forward head posture or a turtle neck syndrome of the user by displaying the visual object.

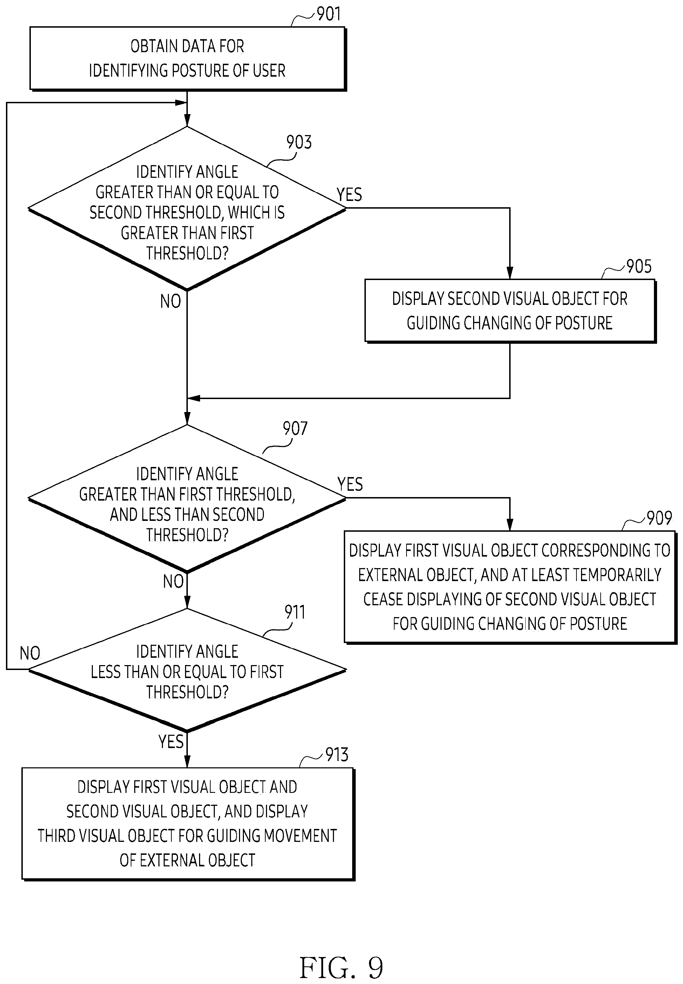

FIG. 9 illustrates an example of a flowchart with respect to an operation of a wearable device according to an embodiment. A wearable device of FIG. 9 may include the wearable device 200 of FIG. 2A and/or FIG. 2B, the wearable device 301 of FIG. 3, FIG. 4, FIG. 5, FIG. 6A, FIG. 6B, FIG. 7A, and/or FIG. 7B, and/or the wearable device of FIG. 8.

Referring to FIG. 9, in operation 901, the wearable device according to an embodiment may obtain data for identifying a posture of a user. For example, the data may be obtained based on a sensor (e.g., the accelerometer, the gyro sensor, and the depth sensor) included in the wearable device. For example, the data may include an electrical signal obtained based on the sensor. Based on the data obtained using the sensor, the wearable device may obtain a parameter associated with the data. Based on obtaining the data and/or the parameter, the wearable device may identify the posture of the user of the wearable device. For example, based on the data and/or the parameter, the wearable device may identify an angle between a first axis (e.g., the first axis 500 of FIG. 5) associated with a body part of the user, and a second axis (e.g., the second axis 505 of FIG. 5) rotatable based on the first axis according to changing of the posture of the user.

In operation 903, according to an embodiment, the wearable device may identify an angle based on the data obtained in the operation 901. The wearable device according to an embodiment may identify whether the angle is greater than or equal to a second threshold (e.g., 11 degrees), which is greater than a first threshold (e.g., -11 degrees). For example, the angle may include the angle between the first axis and the second axis. The wearable device may identify the posture of the user of the wearable device based on identifying the angle. Based on identifying the posture, the wearable device may display a first visual object corresponding to an external object, a second visual object for guiding changing of the posture, and a third visual object for guiding a movement of the external object. An operation for displaying the first visual object, the second visual object, and/or the third visual object will be described later.

In case of identifying the angle, which is greater than or equal to the second threshold (the operation 903-YES), in operation 905, the wearable device according to an embodiment may display the second visual object for guiding changing of the posture of the user. Based on identifying the angle greater than or equal to the second threshold, the wearable device may display the second visual object different from the first visual object corresponding to the external object. For example, the second visual object may be a visual object for guiding the posture of the user. For example, the second visual object may be a visual object for guiding changing of a gaze of the user.

In case of identifying the angle less than the second threshold (the operation 903-NO), in operation 907, the wearable device according to an embodiment may identify whether the angle between the first axis and the second axis is greater than the first threshold, and less than the second threshold bigger than the first threshold. The wearable device may execute a different operation according to the identified angle. For example, the posture of the user corresponding to an angle between the first threshold and the second threshold may be a normal posture.

In case of identifying the angle, which is greater than the first threshold, and less than the second threshold bigger than the first threshold (907-YES), in operation 909, the wearable device according to an embodiment may display the first visual object corresponding to the external object. The wearable device may at least temporarily cease displaying of the second visual object for guiding changing of the posture of the user while displaying the first visual object. For example, the second visual object may include an arrow for inducing changing of the posture of the user. For example, since the posture of the user corresponding to the angle between the first threshold and the second threshold is the normal posture, the wearable device may at least temporarily cease displaying of the second visual object.

In case of not identifying the angle, which is greater the first threshold, and less than the second threshold (907-NO), in operation 911, the wearable device according to an embodiment may identify whether it is an angle less than or equal to the first threshold. For example, the posture of the user corresponding to the angle less than or equal to the first threshold may be a posture in which a neck of the user is tilted back.

In case of identifying the angle, less than or equal to the first threshold (911-YES), in operation 913, the wearable device according to an embodiment may display the first visual object and the second visual object. For example, the first visual object may be the visual object corresponding to the external object. The second visual object may be the visual object for guiding the gaze of the user. While displaying the first visual object and the second visual object, the wearable device according to an embodiment may display the third visual object for guiding the movement of the external object. For example, the third visual object may include a shape such as an arrow to guide the movement of the external object.

As described above, the wearable device according to an embodiment may identify the angle between the first axis and the second axis. The wearable device may identify whether the angle is included in a preset range based on identifying the angle between the first axis and the second axis. The wearable device may display a different screen in the FoV based on the preset range. The wearable device may enhance a user experience of the wearable device by displaying the screen. The wearable device may guide changing of the posture of the user by displaying the screen. The wearable device may prevent a forward head posture or a turtle neck syndrome of the user by guiding changing of the posture of the user.

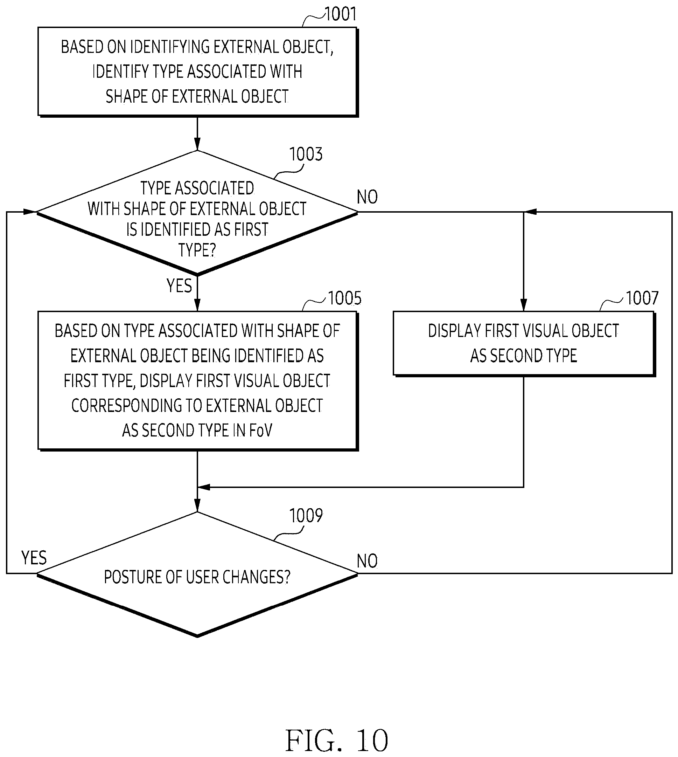

FIG. 10 illustrates an example of a flowchart with respect to an operation of a wearable device according to an embodiment. A wearable device of FIG. 10 may include the wearable device 200 of FIG. 2A and/or FIG. 2B, the wearable device 301 of FIG. 3, FIG. 4, FIG. 5, FIG. 6A, FIG. 6B, FIG. 7A, and/or FIG. 7B, and the wearable device of FIG. 8 and/or FIG. 9.

Referring to FIG. 10, in operation 1001, the wearable device according to an embodiment may identify an external object. Based on identifying the external object, the wearable device may identify a type associated with a shape of the external object. For example, within the shape identifying the external object, the type may be associated with an angle of corners of the shape. For example, the wearable device may identify the type of the external object according to whether the angle is a preset angle (e.g., approximately 90 degrees).

In operation 1003, the wearable device according to an embodiment may identify whether the type associated with the shape of the external object is a first type. For example, a shape of the first type may be a shape in which the angle of the corners is not identified as the preset angle. For example, the shape of the external object may be associated with an ROI including at least a portion of the external object. For example, the wearable device may identify the type associated with the shape of the external object based on the ROI.

In case that the type associated with the shape of the external object is identified as the first type (1003-YES), in operation 1005, the wearable device according to an embodiment may display a first visual object corresponding to the external object in a FoV. The wearable device may display the first visual object as a visual object of a second type. For example, an angle of corners of the visual object of the second type may be the preset angle. For example, the wearable device may change and display an image obtained based on the external object into the visual object of the second type. For example, the wearable device may obtain an image including the external object using a camera. The wearable device may change (or correct) the image, and convert the image into the visual object of the second type. The wearable device may display the visual object of the second type in an area including a center of the FoV.

In operation 1009, the wearable device according to an embodiment may identify a posture of a user while displaying the first visual object of the second type. For example, the wearable device may identify whether the posture of the user changes while displaying the first visual object. As the posture of the user is changed, the wearable device may perform a different operation. For example, in case that the posture of the user is changed (1009-YES), the wearable device may execute the operation 1003. Based on identifying that the posture of the user is changed, the wearable device may identify the external object again. Based on identifying changing of the posture of the user, the wearable device may obtain the ROI from the external object again. In case that the posture of the user is not changed (1009-NO), in operation 1007, the wearable device according to an embodiment may maintain displaying the first visual object as the second type.

As described above, the wearable device according to an embodiment may identify a type associated with the shape of the external object. The wearable device may identify changing of the posture of the user according to the type. The wearable device may identify the external object again according to changing of the posture. The wearable device may reduce a calculation using a processor of the wearable device, by identifying the external object according to the type.

The user of the wearable device may look the external object in a state of wearing the wearable device. In the state of looking at the external object, the user of the wearable device may look at the external object in a bent posture. When looking at the external object in the bent posture, a large load may be applied to a neck of the user, which may cause a forward head posture.

As described above, a wearable device 301 according to an embodiment may comprise a sensor 430, a camera 420, a display 410, and a processor 120. The processor 120 may be configured to obtain, in a state that the wearable device 301 is worn by a user 330, an image using the camera 420. The processor 120 may be configured to identify a posture of the user 330 based on data of the sensor 430. The processor 120 may be configured to, based on identifying the posture of the user 330, which is a first preset posture, obtain a first visual object corresponding to an external object 320 using the camera 420, and display a second visual object 710 or 790 for guiding changing of the posture within a FoV. The processor 120 may be configured to, based on identifying that the posture of the user is changed from the first preset posture to a second preset posture, display the obtained first visual object in an area including a center of the FoV.

As described above, a wearable device according to an embodiment may help, when a user views an external object, the user to view the external object in a correct posture. For example, the wearable device may identify the posture of the user when viewing the external object. The wearable device may perform a guide for changing the posture of the user, based on identifying the posture of the user.

The processor 120 according to an embodiment may be configured to display, in at least a portion of the FoV, a third visual object 720 or 770 for indicating a parameter associated with the data of the sensor 430.

The processor 120 according to an embodiment may be configured to identify, based on obtaining the data of the sensor 430, which is an accelerometer 431, the posture of the user 330.

The processor 120 according to an embodiment may be configured to identify, based on the accelerometer 431, an angle between a first axis 500 associated with a body part of the user 330, and a second axis 505 rotatable based on the first axis 500 according to changing of the posture of the user 330. The processor 120 may be configured to identify, based on identifying the angle, the posture of the user 330.

The processor 120 according to an embodiment may be configured to identify, based on identifying the angle, which is greater than a first threshold, and less than a second threshold bigger than the first threshold, the first preset posture.

The processor 120 according to an embodiment may be configured to identify, based on identifying the angle less than or equal to the first threshold, the second preset posture. The processor 120 may be configured to display, based on identifying the second preset posture, while displaying the second visual object 710 or 790, the third visual object 720 or 770 for guiding a movement of the external object 320 in the FoV.

The processor 120 according to an embodiment may be configured to identify, based on identifying the angle greater than or equal to the second threshold, a third preset posture.

The processor 120 according to an embodiment may be configured to display a fourth visual object, which is a virtual object corresponding to the posture of the user 330.

The processor 120 according to an embodiment may be configured to identify, based on the data of the sensor 430, which is a depth sensor 432, a distance between the external object 320 and the wearable device 301.

The processor 120 according to an embodiment may be configured to identify, based on the distance identified as less than a preset distance, the third preset posture.

The wearable device 301 according to an embodiment may further comprise communication circuitry 440. The processor 120 may be configured to, in a state that a communication link of the wearable device 301 and the external object 320 is established through the communication circuitry 440, receive information to display a screen of the external object 320 from the external object 320. The processor 120 may be configured to display, based on the information, the screen in the FoV.

The wearable device 301 according to an embodiment may further comprise a haptic sensor 433. The processor 120 may be configured to guide, based on identifying the preset posture, changing of the posture using the haptic sensor 433.

As described above, a method of a wearable device 301 according to an embodiment may comprise obtaining, in a state that the wearable device 301 is wom by a user 330, an image using a camera 420. The method of the wearable device 301 may comprise, in the state, identifying a posture of the user 330 based on data of a sensor. The method of the wearable device 301 may comprise, based on identifying the posture of the user 330, which is a first preset posture, obtaining a first visual object 620 corresponding to an external object 320 using the camera 420, and displaying a second visual object 710 or 790 for guiding changing of the posture within a field-of-view (FoV). The method of the wearable device 301 may comprise, based on identifying that the posture of the user 330 is changed from the first preset posture to a second preset posture, displaying the obtained first visual object 620 in an area including a center of the FoV.

The method according to an embodiment may comprise displaying, in at least a portion of the FoV, a third visual object 720 or 770 for indicating a parameter associated with the data of the sensor.

The method according to an embodiment may comprise identifying, based on obtaining the data of the sensor, which is an accelerometer 431, the posture of the user 330.

The method according to an embodiment may comprise identifying, based on the accelerometer 431, an angle between a first axis 500 associated with a body part of the user 330, and a second axis 505 rotatable based on the first axis 500 according to changing of the posture of the user 330. The method may comprise identifying, based on identifying the angle, the posture of the user 330.

The method according to an embodiment may comprise identifying, based on identifying the angle, which is greater than a first threshold, and less than a second threshold bigger than the first threshold, the first preset posture.

The method according to an embodiment may comprise identifying, based on identifying the angle less than or equal to the first threshold, the second preset posture. The method may comprise displaying, based on identifying the second preset posture, while displaying the second visual object 710 or 790, the third visual object 720 or 770 for guiding a movement of the external object 320 in the FoV.

The method according to an embodiment may comprise identifying, based on identifying the angle greater than or equal to the second threshold, a third preset posture.

The method according to an embodiment may comprise displaying a fourth visual object, which is a virtual object corresponding to the posture of the user 330.

The method according to an embodiment may comprise identifying, based on the data of the sensor, which is a time-of-flight (ToF) sensor, a distance between the external object 320 and the wearable device 301.

The method according to an embodiment may comprise identifying, based on the distance identified as less than a preset distance, the third preset posture.

The method according to an embodiment may comprise, in a state that a communication link of the wearable device 301 and the external object 320 is established through communication circuitry 440, receiving information to display a screen of the external object 320 from the external object 320. The method may comprise displaying, based on the information, the screen in the FoV.

The method according to an embodiment may comprise guiding, based on identifying the preset posture, changing of the posture using the haptic sensor 433.

As described above, in a computer-readable storage medium storing one or more programs according to an embodiment, the one or more programs, when executed by a processor 120 of a wearable device 301, may cause the processor 120 of the wearable device 301 to obtain, in a state that the wearable device 301 is worn by a user 330, an image using a camera 420. The one or more programs, when executed by the processor 120 of the wearable device 301, may cause the processor 120 of the wearable device 301 to, in the state, identify a posture of the user 330 based on data of a sensor. The one or more programs, when executed by the processor 120 of the wearable device 301, may cause the processor 120 of the wearable device 301 to, based on identifying the posture of the user 330, which is a first preset posture, obtain a first visual object 620 corresponding to an external object 320 using the camera 420, and display a second visual object 710 or 790 for guiding changing of the posture within a FoV. The one or more programs, when executed by the processor 120 of the wearable device 301, may cause the processor 120 of the wearable device 301 to, based on identifying that the posture of the user 330 is changed from the first preset posture to a second preset posture, display the obtained first visual object 620 in an area including a center of the FoV.

The one or more programs according to an embodiment, when executed by the processor 120 of the wearable device 301, may cause the processor 120 of the wearable device 301 to display, in at least a portion of the FoV, a third visual object 720 or 770 for indicating a parameter associated with the data of the sensor.

The one or more programs according to an embodiment, when executed by the processor 120 of the wearable device 301, may cause the processor 120 of the wearable device 301 to identify, based on obtaining the data of the sensor 430, which is an accelerometer 431, the posture of the user 330.

The one or more programs according to an embodiment, when executed by the processor 120 of the wearable device 301, may cause the processor 120 of the wearable device 301 to identify, based on the accelerometer 431, an angle between a first axis 500 associated with a body part of the user 330, and a second axis 505 rotatable based on the first axis 500 according to changing of the posture of the user 330. The one or more programs, when executed by the processor 120 of the wearable device 301, may cause the processor 120 of the wearable device 301 to identify, based on identifying the angle, the posture of the user 330.

The one or more programs according to an embodiment, when executed by the processor 120 of the wearable device 301, may cause the processor 120 of the wearable device 301 to identify, based on identifying the angle less than or equal to the first threshold, the second preset posture. The one or more programs, when executed by the processor 120 of the wearable device 301, may cause the processor 120 of the wearable device 301 to display, based on identifying the second preset posture, while displaying the second visual object 710 or 790, the third visual object 720 or 770 for guiding a movement of the external object 320 in the FoV.

The one or more programs according to an embodiment, when executed by the processor 120 of the wearable device 301, may cause the processor 120 of the wearable device 301 to identify, based on identifying the angle less than or equal to the first threshold, the second preset posture. The one or more programs, when executed by the processor 120 of the wearable device 301, may cause the processor 120 of the wearable device 301 to display, based on identifying the second preset posture, while displaying the second visual object 710 or 790, the third visual object 720 or 770 for guiding the movement of the external object 320 in the FoV.

The one or more programs according to an embodiment, when executed by the processor 120 of the wearable device 301, may cause the processor 120 of the wearable device 301 to identify, based on identifying the angle greater than or equal to the second threshold, a third preset posture.

The one or more programs according to an embodiment, when executed by the processor 120 of the wearable device 301, may cause the processor 120 of the wearable device 301 to display a fourth visual object, which is a virtual object corresponding to the posture of the user 330.

The one or more programs according to an embodiment, when executed by the processor 120 of the wearable device 301, may cause the processor 120 of the wearable device 301 to identify, based on the data of the sensor 430, which is a depth sensor 432, a distance between the external object 320 and the wearable device 301.

The one or more programs according to an embodiment, when executed by the processor 120 of the wearable device 301, may cause the processor 120 of the wearable device 301 to identify, based on the distance identified as less than a preset distance, the third preset posture.

The one or more programs according to an embodiment, when executed by the processor 120 of the wearable device 301, may receive, in a state that a communication link of the wearable device 301 and the external object 320 is established through communication circuitry 440, information to display a screen of the external object 320 from the external object 320. The one or more programs, when executed by the processor 120 of the wearable device 301, may cause the processor 120 of the wearable device 301 to display, based on the information, the screen in the FoV.

The one or more programs according to an embodiment, when executed by the processor 120 of the wearable device 301, may cause the processor 120 of the wearable device 301 to guide, based on identifying the preset posture, changing of the posture using a haptic sensor 433.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments . With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added. The invention is defined by the appended claims.

## Claims

1. A wearable device (301) comprising:
a sensor (300);
one or more cameras (420);
one or more another cameras (423) configured to be arranged in front of eyes of a user;
a display (410);
memory comprising one or more storage media storing instructions; and
at least one processor (120) comprising processing circuitry, wherein instructions, when executed by the at least one processor (120), cause the wearable device (301) to:
identify, via the one or more another cameras (423) a gaze of the eyes of the user;
detect, using the one or more cameras (420), an external object in which the gaze faces within a field of view (FOV) of the one or more cameras (420);
identify, using the sensor, a posture of the wearable device (301);
based on the posture of the wearable device (301) being identified as a first posture of the wearable device (301) different from a reference posture of the wearable device (301) while the external object is detected, display, via the display (410), information (710, 790) for a guide to the reference posture of the wearable device (301); and
based on the posture of the wearable device (301) being identified as a second posture of the wearable device (301) corresponding to the reference posture of the wearable device (301) while the external object is detected, display, via the display (410), an image obtained using the one or more cameras (420) with respect to the external object.

2. The wearable device (301) of claim 1, wherein instructions, when executed by the at least one processor (120), cause the wearable device (301) to:
based on the posture of the wearable device (301) being identified as the second posture of the wearable device (301) while the external object is detected, concurrently display, via the display (410), the image and another information (710, 790) for a guide to move the external object.

3. The wearable device (301) of claim 1,
wherein instructions, when executed by the at least one processor (120), cause the wearable device (301) to:
based on the posture of the wearable device (301) being identified as the first posture of the wearable device (301) while the external object is detected, concurrently display, via the display (410), the information (710, 790) and another information indicating the first posture of the wearable device (301).

4. The wearable device (301) of claim 1, wherein the image including a visual object corresponding to the external object positioned within a central area of the image.

5. The wearable device (301) of claim 1, wherein instructions, when executed by the at least one processor (120), cause the wearable device (301) to:
identify, via the sensor (300), an angle between a first axis and a second axis rotatable with respect to the first axis in accordance with changing of the posture of the wearable device (301); and
identify the posture of the wearable device (301).

6. The wearable device (301) of claim 5, wherein instructions, when executed by the at least one processor (120), cause the wearable device (301) to:
based on the angle within a reference range, identify the posture of the wearable device (301) as the first posture of the wearable device (301).

7. The wearable device (301) of claim 5, wherein instructions, when executed by the at least one processor (120), cause the wearable device (301) to:
based on the angle out of the reference range, identify the posture of the wearable device (301) as the second posture of the wearable device (301).

8. The wearable device (301) of claim 1, further comprising one or more another cameras,
wherein instructions, when executed by the at least one processor (120), cause the wearable device (301) to:
identify, via the one or more another cameras, a gaze of eyes of a user wearing the wearable device (301);
based on identifying, using the one or more cameras (420), the gaze of the user facing the external object, detect the external object positioned in the FOV of the one or more cameras (420).

9. The wearable device (301) of claim 1, further comprising another sensor,
wherein instructions, when executed by the at least one processor (120), cause the wearable device (301) to:
identify, using the another sensor, a depth value of the external object;
based on the depth value of the external object being identified as lower than a reference value, detect the external object positioned in the FOV of the one or more cameras (420).

10. The wearable device (301) of claim 1, further comprising communication circuitry, wherein instructions, when executed by the at least one processor (120), cause the wearable device (301) to:
based on the posture of the wearable device (301) being identified as the second posture of the wearable device (301) while the external object is detected, request, using the communication circuitry, via a communication link between the wearable device (301) and the external object, from the external object, another information being displayed on a screen on the external object;
receive, using the communication circuitry, via the communication link, from the external object, the another information; and
display, via the display (410), the image using the another information.

11. A method executed in a wearable device (301), comprising a sensor (300), one or more cameras (420), and a display (410), the method comprising:
identifying, via the one or more another cameras (423) a gaze of the eyes of the user;
detecting, using the one or more cameras (420), an external object in which the gaze faces within a field of view (FOV) of the one or more cameras (420);
identifying, using the sensor, a posture of the wearable device (301);
based on the posture of the wearable device (301) being identified as a first posture of the wearable device (301) different from a reference posture of the wearable device (301) while the external object is detected, displaying, via the display (410), information (710, 790) for a guide to the reference posture of the wearable device (301); and
based on the posture of the wearable device (301) being identified as a second posture of the wearable device (301) corresponding to the reference posture of the wearable device (301) while the external object is detected, display, via the display (410), an image obtained using the one or more cameras (420) with respect to the external object.

12. The method of claim 11, the method comprising:
based on the posture of the wearable device (301) being identified as the second posture of the wearable device (301) while the external object is detected, concurrently displaying, via the display (410), the image and another information (710, 790) for a guide to move the external object.

13. The method of claim 11, the method comprising:
based on the posture of the wearable device (301) being identified as the first posture of the wearable device (301) while the external object is detected, concurrently displaying, via the display (410), the information (710, 790) and another information indicating the first posture of the wearable device (301).

14. The method of claim 11, wherein the image including a visual object corresponding to the external object positioned within a central area of the image.

15. The method of claim 11, the method comprising:
identifying, via the sensor (300), an angle between a first axis and a second axis rotatable with respect to the first axis in accordance with changing of the posture of the wearable device (301); and
identifying the posture of the wearable device (301).

## Patentansprüche

1. Tragbare Vorrichtung (301), umfassend:
einen Sensor (300);
eine oder mehrere Kameras (420);
eine oder mehrere andere Kameras (423), die dazu konfiguriert sind, vor Augen eines Benutzers angeordnet zu werden;
eine Anzeige (410);
Speicher, der ein oder mehrere Speichermedien umfasst, die Anweisungen speichern; und
mindestens einen Prozessor (120), der eine Verarbeitungsschaltung umfasst, wobei Anweisungen, wenn sie durch den mindestens einen Prozessor (120) ausgeführt werden, Folgendes durch die tragbare Vorrichtung (301) bewirken:
Identifizieren eines Blicks der Augen des Benutzers über die eine oder die mehreren anderen Kameras (423);
Detektieren eines externen Objekts, auf das der Blick innerhalb eines Sichtfelds (FOV) der einen oder der mehreren Kameras (420) gerichtet ist, unter Verwendung der einen oder der mehreren Kameras (420);
Identifizieren einer Haltung der tragbaren Vorrichtung (301) unter Verwendung des Sensors;
basierend darauf, dass die Haltung der tragbaren Vorrichtung (301) als eine erste Haltung der tragbaren Vorrichtung (301) identifiziert wird, die sich von einer Referenzhaltung der tragbaren Vorrichtung (301) unterscheidet, während das externe Objekt detektiert wird, Anzeigen von Informationen (710, 790) für eine Führung zu der Referenzhaltung der tragbaren Vorrichtung (301) über die Anzeige (410); und
basierend darauf, dass die Haltung der tragbaren Vorrichtung (301) als eine zweite Haltung der tragbaren Vorrichtung (301) identifiziert wird, die der Referenzhaltung der tragbaren Vorrichtung (301) entspricht, während das externe Objekt detektiert wird, Anzeigen eines Bilds, das unter Verwendung der einen oder der mehreren Kameras (420) in Bezug auf das externe Objekt erlangt wird, über die Anzeige (410).

2. Tragbare Vorrichtung (301) nach Anspruch 1, wobei Anweisungen, wenn sie durch den mindestens einen Prozessor (120) ausgeführt werden, Folgendes durch die tragbare Vorrichtung (301) bewirken:
basierend darauf, dass die Haltung der tragbaren Vorrichtung (301) als die zweite Haltung der tragbaren Vorrichtung (301) identifiziert wird, während das externe Objekt detektiert wird, gleichzeitiges Anzeigen des Bilds und anderer Informationen (710, 790) für eine Führung zum Bewegen des externen Objekts über die Anzeige (410).

3. Tragbare Vorrichtung (301) nach Anspruch 1,
wobei Anweisungen, wenn sie durch den mindestens einen Prozessor (120) ausgeführt werden, Folgendes durch die tragbare Vorrichtung (301) bewirken:
basierend darauf, dass die Haltung der tragbaren Vorrichtung (301) als die erste Haltung der tragbaren Vorrichtung (301) identifiziert wird, während das externe Objekt detektiert wird, gleichzeitiges Anzeigen der Informationen (710, 790) und anderer Informationen, die die erste Haltung der tragbaren Vorrichtung (301) angeben, über die Anzeige (410).

4. Tragbare Vorrichtung (301) nach Anspruch 1, wobei das Bild einschließlich eines visuellen Objekts, das dem externen Objekt entspricht, das innerhalb einer zentralen Fläche des Bilds positioniert ist.

5. Tragbare Vorrichtung (301) nach Anspruch 1, wobei Anweisungen, wenn sie durch den mindestens einen Prozessor (120) ausgeführt werden, Folgendes durch die tragbare Vorrichtung (301) bewirken:
Identifizieren eines Winkels zwischen einer ersten Achse und einer zweiten Achse, die in Bezug auf die erste Achse gemäß einem Ändern der Haltung der tragbaren Vorrichtung (301) drehbar ist, über den Sensor (300); und
Identifizieren der Haltung der tragbaren Vorrichtung (301).

6. Tragbare Vorrichtung (301) nach Anspruch 5, wobei Anweisungen, wenn sie durch den mindestens einen Prozessor (120) ausgeführt werden, Folgendes durch die tragbare Vorrichtung (301) bewirken:
basierend auf dem Winkel innerhalb eines Referenzbereichs Identifizieren der Haltung der tragbaren Vorrichtung (301) als die erste Haltung der tragbaren Vorrichtung (301).

7. Tragbare Vorrichtung (301) nach Anspruch 5, wobei Anweisungen, wenn sie durch den mindestens einen Prozessor (120) ausgeführt werden, Folgendes durch die tragbare Vorrichtung (301) bewirken:
basierend auf dem Winkel außerhalb des Referenzbereichs Identifizieren der Haltung der tragbaren Vorrichtung (301) als die zweite Haltung der tragbaren Vorrichtung (301).

8. Tragbare Vorrichtung (301) nach Anspruch 1, ferner umfassend eine oder mehrere andere Kameras,
wobei Anweisungen, wenn sie durch den mindestens einen Prozessor (120) ausgeführt werden, Folgendes durch die tragbare Vorrichtung (301) bewirken:
Identifizieren eines Blicks von Augen eines Benutzers, der die tragbare Vorrichtung (301) trägt, über die eine oder die mehreren anderen Kameras;
basierend darauf, dass unter Verwendung der einen oder der mehreren Kameras (420) identifiziert wird, dass der Blick des Benutzers auf das externe Objekt gerichtet ist, Detektieren des externen Objekts, das in dem FOV der einen oder der mehreren Kameras (420) positioniert ist.

9. Tragbare Vorrichtung (301) nach Anspruch 1, ferner umfassend einen anderen Sensor,
wobei Anweisungen, wenn sie durch den mindestens einen Prozessor (120) ausgeführt werden, Folgendes durch die tragbare Vorrichtung (301) bewirken:
Identifizieren eines Tiefenwerts des externen Objekts unter Verwendung des anderen Sensors;
basierend darauf, dass der Tiefenwert des externen Objekts als niedriger als ein Referenzwert identifiziert wird, Detektieren des externen Objekts, das in dem FOV der einen oder der mehreren Kameras (420) positioniert ist.

10. Tragbare Vorrichtung (301) nach Anspruch 1, ferner umfassend eine Kommunikationsschaltung, wobei Anweisungen, wenn sie durch den mindestens einen Prozessor (120) ausgeführt werden, Folgendes durch die tragbare Vorrichtung (301) bewirken:
basierend darauf, dass die Haltung der tragbaren Vorrichtung (301) als die zweite Haltung der tragbaren Vorrichtung (301) identifiziert wird, während das externe Objekt detektiert wird, Anfordern von anderen Informationen, die auf einem Bildschirm auf dem externen Objekt angezeigt werden, von dem externen Objekt unter Verwendung der Kommunikationsschaltung über eine Kommunikationsverbindung zwischen der tragbaren Vorrichtung (301) und dem externen Objekt;
Empfangen der anderen Informationen von dem externen Objekt unter Verwendung der Kommunikationsschaltung über die Kommunikationsverbindung; und
Anzeigen des Bilds unter Verwendung der anderen Informationen über die Anzeige (410).

11. Verfahren, ausgeführt in einer tragbaren Vorrichtung (301), umfassend einen Sensor (300), eine oder mehrere Kameras (420) und eine Anzeige (410), wobei das Verfahren Folgendes umfasst:
Identifizieren eines Blicks der Augen des Benutzers über die eine oder die mehreren anderen Kameras (423);
Detektieren eines externen Objekts, auf das der Blick innerhalb eines Sichtfelds (FOV) der einen oder der mehreren Kameras (420) gerichtet ist, unter Verwendung der einen oder der mehreren Kameras (420);
Identifizieren einer Haltung der tragbaren Vorrichtung (301) unter Verwendung des Sensors;
basierend darauf, dass die Haltung der tragbaren Vorrichtung (301) als eine erste Haltung der tragbaren Vorrichtung (301) identifiziert wird, die sich von einer Referenzhaltung der tragbaren Vorrichtung (301) unterscheidet, während das externe Objekt detektiert wird, Anzeigen von Informationen (710, 790) für eine Führung zu der Referenzhaltung der tragbaren Vorrichtung (301) über die Anzeige (410); und
basierend darauf, dass die Haltung der tragbaren Vorrichtung (301) als eine zweite Haltung der tragbaren Vorrichtung (301) identifiziert wird, die der Referenzhaltung der tragbaren Vorrichtung (301) entspricht, während das externe Objekt detektiert wird, Anzeigen eines Bilds, das unter Verwendung der einen oder der mehreren Kameras (420) in Bezug auf das externe Objekt erlangt wird, über die Anzeige (410).

12. Verfahren nach Anspruch 11, wobei das Verfahren Folgendes umfasst:
basierend darauf, dass die Haltung der tragbaren Vorrichtung (301) als die zweite Haltung der tragbaren Vorrichtung (301) identifiziert wird, während das externe Objekt detektiert wird, gleichzeitiges Anzeigen des Bilds und anderer Informationen (710, 790) für eine Führung zum Bewegen des externen Objekts über die Anzeige (410).

13. Verfahren nach Anspruch 11, wobei das Verfahren Folgendes umfasst:
basierend darauf, dass die Haltung der tragbaren Vorrichtung (301) als die erste Haltung der tragbaren Vorrichtung (301) identifiziert wird, während das externe Objekt detektiert wird, gleichzeitiges Anzeigen der Informationen (710, 790) und anderer Informationen, die die erste Haltung der tragbaren Vorrichtung (301) angeben, über die Anzeige (410).

14. Verfahren nach Anspruch 11, wobei das Bild einschließlich eines visuellen Objekts, das dem externen Objekt entspricht, das innerhalb einer zentralen Fläche des Bilds positioniert ist.

15. Verfahren nach Anspruch 11, wobei das Verfahren Folgendes umfasst:
Identifizieren eines Winkels zwischen einer ersten Achse und einer zweiten Achse, die in Bezug auf die erste Achse gemäß einem Ändern der Haltung der tragbaren Vorrichtung (301) drehbar ist, über den Sensor (300); und
Identifizieren der Haltung der tragbaren Vorrichtung (301).

## Revendications

1. Dispositif à porter sur soi (301), comprenant :
un capteur (300) ;
une ou plusieurs caméras (420) ;
une ou plusieurs autres caméras (423) configurées pour être agencées devant les yeux d'un utilisateur ;
un dispositif d'affichage (410) ;
une mémoire comprenant un ou plusieurs supports de stockage stockant des instructions ; et
au moins un processeur (120) comprenant un circuit de traitement, dans lequel des instructions, lorsqu'elles sont exécutées par l'au moins un processeur (120), amènent le dispositif à porter sur soi (301) à :
identifier, via l'une ou plusieurs autres caméras (423), un regard des yeux de l'utilisateur ;
détecter, à l'aide de l'une ou plusieurs caméras (420), un objet externe vers lequel est orienté le regard dans un champ de vision (FOV) de l'une ou plusieurs caméras (420) ;
identifier, à l'aide du capteur, une posture du dispositif à porter sur soi (301) ;
sur la base du fait que la posture du dispositif à porter sur soi (301) soit identifiée comme une première posture du dispositif à porter sur soi (301) différente d'une posture de référence du dispositif à porter sur soi (301) pendant que l'objet externe est détecté, afficher, via le dispositif d'affichage (410), des informations (710, 790) de guidage vers la posture de référence du dispositif à porter sur soi (301) ; et
sur la base du fait que la posture du dispositif à porter sur soi (301) soit identifiée comme une seconde posture du dispositif à porter sur soi (301) correspondant à la posture de référence du dispositif à porter sur soi (301) pendant que l'objet externe est détecté, afficher, via le dispositif d'affichage (410), une image obtenue à l'aide de l'une ou plusieurs caméras (420) par rapport à l'objet externe.

2. Dispositif à porter sur soi (301) selon la revendication 1, dans lequel les instructions, lorsqu'elles sont exécutées par l'au moins un processeur (120), amènent le dispositif à porter sur soi (301) à :
sur la base du fait que la posture du dispositif à porter sur soi (301) soit identifiée comme la seconde posture du dispositif à porter sur soi (301) pendant que l'objet externe est détecté, afficher simultanément, via le dispositif d'affichage (410), l'image et d'autres informations (710, 790) de guidage pour déplacer l'objet externe.

3. Dispositif à porter sur soi (301) selon la revendication 1,
dans lequel les instructions, lorsqu'elles sont exécutées par l'au moins un processeur (120), amènent le dispositif à porter sur soi (301) à :
sur la base du fait que la posture du dispositif à porter sur soi (301) soit identifiée comme la première posture du dispositif à porter sur soi (301) pendant que l'objet externe est détecté, afficher simultanément, via le dispositif d'affichage (410), les informations (710, 790) et d'autres informations indiquant la première posture du dispositif à porter sur soi (301).

4. Dispositif à porter sur soi (301) selon la revendication 1, dans lequel l'image comprend un objet visuel correspondant à l'objet externe positionné dans une zone centrale de l'image.

5. Dispositif à porter sur soi (301) selon la revendication 1, dans lequel les instructions, lorsqu'elles sont exécutées par l'au moins un processeur (120), amènent le dispositif à porter sur soi (301) à :
identifier, via le capteur (300), un angle entre un premier axe et un second axe pouvant tourner par rapport au premier axe en fonction du changement de la posture du dispositif à porter sur soi (301) ; et
identifier la posture du dispositif à porter sur soi (301).

6. Dispositif à porter sur soi (301) selon la revendication 5, dans lequel les instructions, lorsqu'elles sont exécutées par l'au moins un processeur (120), amènent le dispositif à porter sur soi (301) à :
sur la base de l'angle compris dans une plage de référence, identifier la posture du dispositif à porter sur soi (301) comme la première posture du dispositif à porter sur soi (301).

7. Dispositif à porter sur soi (301) selon la revendication 5, dans lequel les instructions, lorsqu'elles sont exécutées par l'au moins un processeur (120), amènent le dispositif à porter sur soi (301) à :
sur la base de l'angle en dehors de la plage de référence, identifier la posture du dispositif à porter sur soi (301) comme la seconde posture du dispositif à porter sur soi (301).

8. Dispositif à porter sur soi (301) selon la revendication 1, comprenant en outre une ou plusieurs autres caméras,
dans lequel les instructions, lorsqu'elles sont exécutées par l'au moins un processeur (120), amènent le dispositif à porter sur soi (301) à :
identifier, via l'une ou plusieurs autres caméras, un regard des yeux d'un utilisateur portant le dispositif à porter sur soi (301) ;
sur la base de l'identification, à l'aide de l'une ou plusieurs caméras (420), que le regard de l'utilisateur est orienté vers l'objet externe, détecter l'objet externe positionné dans le FOV de l'une ou plusieurs caméras (420).

9. Dispositif à porter sur soi (301) selon la revendication 1, comprenant en outre un autre capteur,
dans lequel les instructions, lorsqu'elles sont exécutées par l'au moins un processeur (120), amènent le dispositif à porter sur soi (301) à :
identifier, à l'aide de l'autre capteur, une valeur de profondeur de l'objet externe ;
sur la base du fait que la valeur de profondeur de l'objet externe soit identifiée comme étant inférieure à une valeur de référence, détecter l'objet externe positionné dans le FOV de l'une ou plusieurs caméras (420).

10. Dispositif à porter sur soi (301) selon la revendication 1, comprenant en outre un circuit de communication, dans lequel les instructions, lorsqu'elles sont exécutées par l'au moins un processeur (120), amènent le dispositif à porter sur soi (301) à :
sur la base du fait que la posture du dispositif à porter sur soi (301) soit identifiée comme la seconde posture du dispositif à porter sur soi (301) pendant que l'objet externe est détecté, demander, à l'aide du circuit de communication, via une liaison de communication entre le dispositif à porter sur soi (301) et l'objet externe, à partir de l'objet externe, d'autres informations étant affichées sur un écran sur l'objet externe ;
recevoir, en utilisant le circuit de communication, via la liaison de communication, à partir de l'objet externe, les autres informations ; et
afficher, via le dispositif d'affichage (410), l'image à l'aide des autres informations.

11. Procédé exécuté dans un dispositif à porter sur soi (301), comprenant un capteur (300), une ou plusieurs caméras (420) et un dispositif d'affichage (410), le procédé comprenant :
l'identification, via l'une ou plusieurs autres caméras (423), d'un regard des yeux de l'utilisateur ;
la détection, à l'aide de l'une ou plusieurs caméras (420), d'un objet externe vers lequel est orienté le regard dans un champ de vision (FOV) de l'une ou plusieurs caméras (420) ;
l'identification, à l'aide du capteur, d'une posture du dispositif à porter sur soi (301) ;
sur la base du fait que la posture du dispositif à porter sur soi (301) soit identifiée comme une première posture du dispositif à porter sur soi (301) différente d'une posture de référence du dispositif à porter sur soi (301) pendant que l'objet externe est détecté, l'affichage, via le dispositif d'affichage (410), d'informations (710, 790) de guidage vers la posture de référence du dispositif à porter sur soi (301) ; et
sur la base du fait que la posture du dispositif à porter sur soi (301) soit identifiée comme une seconde posture du dispositif à porter sur soi (301) correspondant à la posture de référence du dispositif à porter sur soi (301) pendant que l'objet externe est détecté, l'affichage, via le dispositif d'affichage (410), d'une image obtenue à l'aide de l'une ou plusieurs caméras (420) par rapport à l'objet externe.

12. Procédé selon la revendication 11, le procédé comprenant :
sur la base du fait que la posture du dispositif à porter sur soi (301) soit identifiée comme la seconde posture du dispositif à porter sur soi (301) pendant que l'objet externe est détecté, l'affichage simultané, via le dispositif d'affichage (410), de l'image et d'autres informations (710, 790) de guidage pour déplacer l'objet externe.

13. Procédé selon la revendication 11, le procédé comprenant :
sur la base du fait que la posture du dispositif à porter sur soi (301) soit identifiée comme la première posture du dispositif à porter sur soi (301) pendant que l'objet externe est détecté, l'affichage simultané, via le dispositif d'affichage (410), des informations (710, 790) et d'autres informations indiquant la première posture du dispositif à porter sur soi (301).

14. Dispositif à porter sur soi selon la revendication 11, dans lequel l'image comprend un objet visuel correspondant à l'objet externe positionné dans une zone centrale de l'image.

15. Procédé selon la revendication 11, le procédé comprenant :
l'identification, via le capteur (300), d'un angle entre un premier axe et un second axe pouvant tourner par rapport au premier axe en fonction du changement de la posture du dispositif à porter sur soi (301) ; et
l'identification de la posture du dispositif à porter sur soi (301).
